# EUROPEAN PATENT SPECIFICATION

(11) **EP 4 154 881 B1**
(45) Date of publication and mention of the grant of the patent: **26.02.2025**
(21) Application number: 21825627.9
(22) Date of filing: 27.05.2021
(51) Int. Cl.: A61K 31/4439, A61K 31/444, A61K 31/4545, A61K 31/496, A61K 31/5377, A61P 31/14, A61P 31/16, A61P 31/20, A61P 31/22, A61P 1/00, A61P 1/12, A61P 7/06, A61P 11/00, A61P 17/00, A61P 35/02, A61P 43/00

(54) **APPLICATIONS OF HPK1 KINASE INHIBITOR IN PREVENTING AND/OR TREATING ANIMAL PATHOGEN INFECTION**
ANWENDUNGEN EINES HPK1-KINASEHEMMERS BEI DER VORBEUGUNG UND/ODER BEHANDLUNG VON INFEKTIONEN DURCH TIERISCHE PATHOGENE
APPLICATIONS D'UN INHIBITEUR DE KINASE HPK1 DANS LA PRÉVENTION ET/OU LE TRAITEMENT D'UNE INFECTION PAR UN AGENT ZOOPATHOGÈNE

(30) Priority: 16.06.2020 CN 202010546816
(43) Date of publication of application: 29.03.2023
(73) Proprietor: Zhuhai Yufan Biotechnologies Co., Ltd, Zhuhai, Guangdong 519031 (CN)
(72) Inventor: LIN, Xingyu, Zhuhai, Guangdong 519000 (CN); LU, Tingting, Zhuhai, Guangdong 519000 (CN)
(74) Representative: Ipside
(86) International application number: PCT/CN2021/096276
(87) International publication number: WO 2021/254118

(56) References cited:
- EP-A1- 3 782 993
- EP-A1- 4 094 763
- EP-A1- 4 223 752
- WO-A1-2016/188972
- WO-A1-2019/090198
- WO-A1-2019/126505
- WO-A1-2019/196937
- WO-A1-2019/206049
- WO-A1-2019/221601
- WO-A1-2020/255022
- WO-A2-2018/081531
- TRICIA L. MAY-DRACKA ET AL: "Investigating small molecules to inhibit germinal center kinase-like kinase (GLK/MAP4K3) upstream of PKCθ phosphorylation: Potential therapy to modulate T cell dependent immunity", BIOORGANIC & MEDICINAL CHEMISTRY LETTERS, vol. 28, no. 10, 1 June 2018 (2018-06-01), Amsterdam NL, pages 1964 - 1971, XP055644699, ISSN: 0960-894X, DOI: 10.1016/j.bmcl.2018.03.032
- KUMAR, A. RHODES, R. SPYCHALA, J. WILSON, W. BOYKIN, D. TIDWELL, R. DYKSTRA, C. HALL, J. JONES, S. SCHINAZI, R.: "Synthesis of dicationic diarylpyridines as nucleic-acid binding agents", EUROPEAN JOURNAL OF MEDICINAL CHEMISTRY, ELSEVIER, AMSTERDAM, NL, vol. 30, no. 2, 1 January 1995 (1995-01-01), AMSTERDAM, NL , pages 99 - 106, XP004040124, ISSN: 0223-5234, DOI: 10.1016/0223-5234(96)88214-6
- HERNANDEZ SAIRY, QING JING, THIBODEAU REBECCA HONG, DU XIANGNAN, PARK SUMMER, LEE HYANG-MI, XU MIN, OH SOYOUNG, NAVARRO ARMANDO, R: "The Kinase Activity of Hematopoietic Progenitor Kinase 1 Is Essential for the Regulation of T Cell Function", CELL REPORTS, ELSEVIER INC, US, vol. 25, no. 1, 1 October 2018 (2018-10-01), US , pages 80 - 94, XP055881180, ISSN: 2211-1247, DOI: 10.1016/j.celrep.2018.09.012

## Description

### TECHNICAL FIELD

The present invention relates to the technical field of chemical pharmaceutics, and in particular to a HPK1 kinase inhibitor of formula II for use in preventing and/or treating coronaviruses infection in animals.

### BACKGROUND

Viral diseases are a group of diseases doing most serious harm to animals in veterinary practice, and particularly, the prevalence of some highly pathogenic viral diseases often cause enormous economic and mental losses to the breeders. Viral diseases in animals usually spread quickly, feature high morbidity rate and mortality rate, and seriously endanger the health and life of animals. Moreover, some viruses can be transmitted to human beings through animals, threatening the safety of human beings.

Viral diseases in animals can be caused by a wide variety of viruses, for example, viruses being of Herpesviridae, Rhabdoviridae, Reoviridae, Poxviridae, Asfarviridae, Adenoviridae, Parvoviridae, Circoviridae, Orthomyxoviridae, Paramyxoviridae and Coronaviridae. Coronaviruses are a large family of viruses widely present in the nature and are named for their morphological similarity to crown under an electron microscope. They mainly cause respiratory diseases and can infect various mammals, such as pigs, cattle, cats, dogs, minks and camels, and various birds. For example, porcine deltacoronavirus (PDCoV) is a novel porcine enteric coronavirus that can cause diarrhea and vomiting, rapid dehydration and death of organ failure in piglets of 5-15 days old, with morbidity and mortality rates up to 50% to 100%; avian infectious bronchitis is an acute highly contagious respiratory infectious diseases in chicken caused by infectious bronchitis virus and is clinically characterized by dyspnea, rales, cough, mouth breathing and sneezing, a non-nephropathogenic strain that does not cause complication generally leads to a low mortality, but to reduced egg production and quality in laying hens; bovine coronavirus (BCV) is a pathogenic virus of cattle, is generally regarded as an important pathogen of neonatal calf diarrhea at present, and can cause respiratory tract infection in cattle and winter bloody dysentery in adult cattle; the epidemic diarrhea (coronavirus enteritis) in minks, foxes and raccoon dogs is caused by coronavirus except for mink parvovirus enteritis, and infected dogs, foxes, raccoon dogs and minks usually demonstrate hemorrhagic gastroenteritis symptoms and are susceptible to cluster epidemic diarrhea, showing a high spreading speed, a mortality more than 30% and a higher morbidity in animals giving birth in the year is higher than in stud animals.

Although many vaccines against viral diseases have been developed with the development of pharmaceutical technology, virus mutants impose a burden of efficacy evaluation of the vaccines against the emerging viruses as well as a financial burden, and the morbidity and mortality of viral diseases still remain high, leaving a great demand for drugs with good antiviral efficacy to the field of veterinary.

WO2019/090198 discloses isofuranone compounds useful as HPK1 inhibitors, and describes that these compounds are useful in the treatment of viral infections and proliferative disorders, such as cancer. However, this application fails to disclose experiments to verify that these compounds are used to inhibit viral infection, let alone specific coronavirus infections such as feline infectious peritonitis virus infection.

### SUMMARY

The invention is set out in the appended set of claims.

The present invention provides a compound, or a pharmaceutically acceptable salt, a solvate or a deuterated compound thereof for use in preventing and/or treating a disease caused by or associated with pathogen infection in an animal;
the compound described above has the following structure: 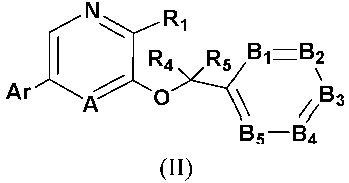 wherein,
A is selected from C and N;
Ar is selected from: 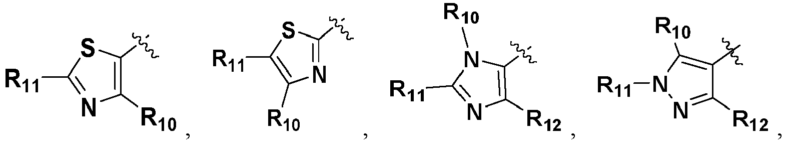 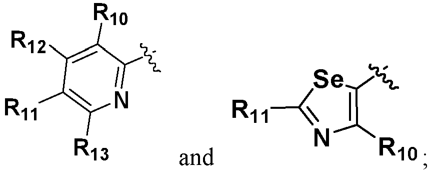
   wherein R₁₀, R₁₂ and R₁₃ are independently selected from: -H, C₁₋₁₀ linear/branched alkyl, -N(C₀₋₁₀ alkyl)(C₀₋₁₀ alkyl), -OC₀₋₁₀ alkyl and C₃₋₁₀ cycloalkyl;
   R₁₁ is selected from: -H, -O heterocycloalkyl, -N heterocycloalkyl, C₁₋₁₀ linear/branched alkyl, C₃₋₁₀ cycloalkyl, -OC₀₋₁₀ alkyl, -N(C₀₋₁₀ alkyl)(C₀₋₁₀ alkyl), -SO₂(C₀₋₁₀ alkyl), -O(C₀₋₁₀ alkyl), -O-phenyl, - S(C₀₋₁₀ alkyl), -N heterocycloaryl, -O heterocycloaryl and -S heterocycloaryl, wherein H on the C atom or heteroatom may be substituted with C₁₋₃ linear alkyl, -N(C₀₋₁₀ alkyl)(C₀₋₁₀ alkyl) or -CF₃;
   R₁ is selected from: -H, halogen, -NO₂, -CN, C₁₋₅ linear/branched alkyl, C₃₋₁₀ cycloalkyl, -N(C₀₋₁₀ alkyl)(C₀₋₁₀ alkyl), -CF₃, -OCF₃, -OCHF₂, -OCH₂F and -OC₀₋₁₀ alkyl;
   R₄ and R₅ are independently selected from: -H, deuterium, halogen, C₁₋₁₀ linear/branched alkyl and deuterated C₁₋₁₀ linear/branched alkyl;
   at least one of B₁, B₂, B₃, B₄ and B₅ is N; or 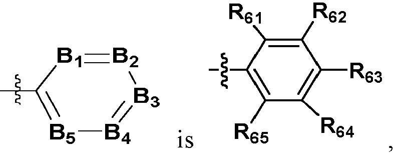 wherein
      R₆₃ is selected from: -H, halogen, -OC₀₋₁₀ alkyl, C₁₋₁₀ linear/branched alkyl, -N(C₀₋₁₀ alkyl)(C₀₋₁₀ alkyl) and C₃₋₁₀ cycloalkyl;
      R₆₁, R₆₄ and R₆₅ are independently selected from: -H, halogen, -CN, -OC₀₋₁₀ alkyl, C₁₋₁₀ linear/branched alkyl, -N(C₀₋₁₀ alkyl)(C₀₋₁₀ alkyl), C₃₋₁₀ cycloalkyl, -C≡C-R₇, -O heterocycloalkyl, -N heterocycloalkyl and C₁₋₅ linear/branched alkyl containing O and N;
      R₆₂ is C≡C-R₇;
      R₇ is selected from H, C₁₋₅ linear/branched alkyl, C₃₋₁₀ cycloalkyl and 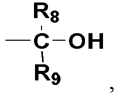 wherein R₈ and R₉ are independently selected from: -H, -CF₃, -CHF₂, -CH₂F, C₁₋₁₀ linear/branched alkyl, -CH=C(C₀₋₁₀ alkyl)(C₀₋₁₀ alkyl), -C≡C(C₀₋₁₀ alkyl), C₃₋₁₀ cycloalkyl, -CO(C₀₋₁₀ alkyl)(C₀₋₁₀ alkyl), -CO(C₀₋₁₀ alkyl)(C₀₋₁₀ alkyl), -CON(C₀₋₁₀ alkyl)(C₀₋₁₀ alkyl), -N(C₀₋₁₀ alkyl)CO(C₀₋₁₀ alkyl)(C₀₋₁₀ alkyl), aromatic five-membered cyclic group and aromatic six-membered cyclic group, or R₈ and R₉, together with carbon atoms between R₈ and R₉, form C₃₋₈ cycloalkyl or C₃₋₈ heterocycloalkyl containing -O- or -S-, C₄₋₉ fused cycloalkyl, C₅₋₁₀ spiro cycloalkyl, C₄₋₉ bridged cycloalkyl, C₃₋₇ cyclolactam, C₃₋₇ cyclic lactone or C₃₋₇ cyclic ketone; and
      the pathogen is a coronavirus.

In one embodiment of the present invention, A described above is C.

In one embodiment of the present invention, the compound described above has the following structure:

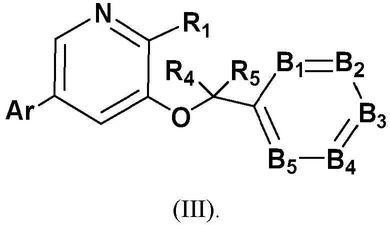

Specifically, R₄ and R₅ described above are independently selected from: -H and C₁₋₁₀ linear/branched alkyl; and more specifically, R₄ and R₅ are independently selected from: -H and C₁₋₃ linear/branched alkyl.

In one embodiment of the present invention, R₄ described above is H or -CH₃.

In one embodiment of the present invention, R₅ described above is H or -CH₃.

Specifically, R₁₀, R₁₂ and R₁₃ described above may be independently selected from: -H, C₁₋₅ linear/branched alkyl and -N(C₀₋₁₀ alkyl)(C₀₋₁₀ alkyl); and more specifically, R₁₀, R₁₂ and R₁₃ may be independently selected from: -H, -CH₃, -CH₂CH₃ and -NH₂.

Specifically, R₁₁ described above may be selected from: -O heterocycloalkyl, -N heterocycloalkyl, - SO₂(C₀₋₃ alkyl), -O-phenyl, -S(C₀₋₄ alkyl), C₃₋₆ cycloalkyl and C₃₋₅ linear/branched alkyl, wherein H on the C atom or heteroatom may be substituted with -CH₃, -NH₂ or -CF₃; and more specifically, R₁₁ described above may be selected from:

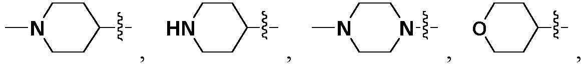

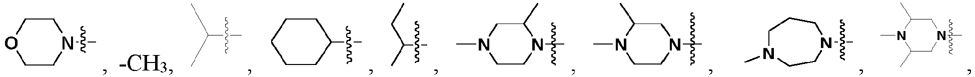

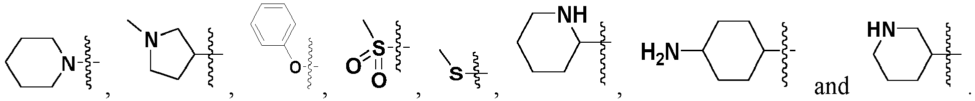

In one embodiment of the present invention, the compound described above has the following structure:

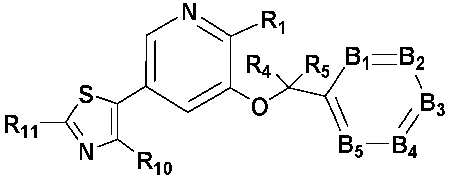

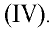

Specifically, R₁ described above may be selected from: -H, halogen, -NO₂, -CN, C₁₋₅ linear/branched alkyl, C₃₋₁₀ cycloalkyl, -N(C₀₋₁₀ alkyl)(C₀₋₁₀ alkyl), -CF₃, -OCF₃, -OCHF₂, -OCH₂F and -OC₀₋₁₀ alkyl; more specifically, R₁ described above may be selected from: -NO₂, -N(C₀₋₁₀ alkyl)(C₀₋₁₀ alkyl), -OC₀₋₁₀ alkyl and -OCF₃; and in one embodiment of the present invention, R₁ described above is -NH₂ or - NO₂.

More specifically, R₆₃ may be selected from: -H, halogen, -OC₀₋₁₀ alkyl and C₁₋₁₀ linear/branched alkyl; and even more specifically, R₆₃ may be selected from: -H, -F and -OCH₃.

More specifically, R₆₁, R₆₄ and R₆₅ described above are independently selected from: -H, halogen, C₁₋₃ linear/branched alkyl, -OC₀₋₃ alkyl, and C₁₋₃ linear/branched alkyl containing N; and even more specifically, R₆₁, R₆₄ and R₆₅ described above are independently selected from: -H, -F, -Cl, -CH₃, - CH₂NH₂, -CN and -OCH₃. In one embodiment of the present invention, R₆₁, R₆₄ and R₆₅ described above are all H.

Specifically, R₇ described above is selected from: H, C₁₋₅ linear/branched alkyl, C₃₋₁₀ cycloalkyl and

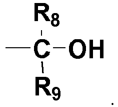

In one embodiment of the present invention, R₆₂ described above is

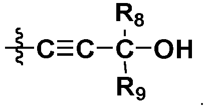

Specifically, R₈ and R₉ described above are independently selected from: -H, -CF₃, -CHF₂, -CH₂F, C₁₋₁₀ linear/branched alkyl, -CH=C(C₀₋₁₀ alkyl)(C₀₋₁₀ alkyl), -C≡C(C₀₋₁₀ alkyl), C₃₋₁₀ cycloalkyl, aromatic five-membered cyclic group and aromatic six-membered cyclic group, or R₈ and R₉, together with carbon atoms between R₈ and R₉, form C₃₋₈ cycloalkyl or C₃₋₈ heterocycloalkyl containing -O or -S, C₄₋₉ fused cycloalkyl, C₅₋₁₀ spiro cycloalkyl, C₄₋₉ bridged cycloalkyl, C₃₋₇ cyclolactam, C₃₋₇ cyclic lactone, or C₃₋₇ cyclic ketone, wherein H on the C atom may be substituted with the following groups: -SO₂, -SO₂N(C₀₋₁₀ alkyl)(C₀₋₁₀ alkyl), -N(C₀₋₁₀ alkyl)SO₂(C₀₋₁₀ alkyl), -CON(C₀₋₁₀ alkyl)(C₀₋₁₀ alkyl), -N(C₀₋₁₀ alkyl)CO(C₀₋₁₀ alkyl), -N(C₀₋₁₀ alkyl)COO(C₀₋₁₀ alkyl), -OCON(C₀₋₁₀ alkyl)(C₀₋₁₀ alkyl), halogen, -CN, -OCH₂F, -OCHF₂, -OCF₃, C₁₋₁₀ linear/branched alkyl, -N(C₀₋₁₀ alkyl)(C₀₋₁₀ alkyl), -OC₀₋₁₀ alkyl, C₃₋₁₀ cycloalkyl, -O heterocycloalkyl, -N heterocycloalkyl, -N heterocycloaryl, -O heterocycloaryl and -S heterocycloaryl, wherein the alkyl moieties may be optionally substituted with one or more of the following groups: -SO₂, -SO₂N(C₀₋₁₀ alkyl)(C₀₋₁₀ alkyl), -N(C₀₋₁₀ alkyl)SO₂(C₀₋₁₀ alkyl), -CON(C₀₋₁₀ alkyl)(C₀₋₁₀ alkyl), -N(C₀₋₁₀ alkyl)CO(C₀₋₁₀ alkyl), -N(C₀₋₁₀ alkyl)COO(C₀₋₁₀ alkyl), -OCON(C₀₋₁₀ alkyl)(C₀₋₁₀ alkyl), halogen, -CN, -OCH₂F, -OCHF₂, -OCF₃, -N(C₀₋₁₀ alkyl)(C₀₋₁₀ alkyl), -OC₀₋₁₀ alkyl, -N heterocycloaryl, -O heterocycloaryl and -S heterocycloaryl.

More specifically, R₈ and R₉ described above are independently selected from: -H, -CF₃, -CHF₂, - CH₂F, C₁₋₁₀ linear/branched alkyl, -CH=C(C₀₋₁₀ alkyl)(C₀₋₁₀ alkyl), C₃₋₁₀ cycloalkyl and aromatic six-membered cyclic group, or R₈ and R₉, together with carbon atoms between R₈ and R₉, form C₃₋₈ cycloalkyl, C₄₋₇ fused cycloalkyl, C₅₋₉ spiro cycloalkyl, C₄₋₉ bridged cycloalkyl, C₃₋₇ cyclolactam, C₃₋₇ cyclic lactone, or C₃₋₇ cyclic ketone, wherein H on the C atom may be substituted with the following groups: -SO₂, -SO₂N(C₀₋₁₀ alkyl)(C₀₋₁₀ alkyl), -N(C₀₋₁₀ alkyl)SO₂(C₀₋₁₀ alkyl), -CON(C₀₋₁₀ alkyl)(C₀₋₁₀ alkyl), -N(C₀₋₁₀ alkyl)CO(C₀₋₁₀ alkyl), -N(C₀₋₁₀ alkyl)COO(C₀₋₁₀ alkyl), -OCON(C₀₋₁₀ alkyl)(C₀₋₁₀ alkyl), halogen, -CN, -OCH₂F, -OCHF₂, -OCF₃, C₁₋₁₀ linear/branched alkyl, -N(C₀₋₁₀ alkyl)(C₀₋₁₀ alkyl), -OC₀₋₁₀ alkyl, C₃₋₁₀ cycloalkyl, -O heterocycloalkyl, -N heterocycloalkyl, -N heterocycloaryl, - O heterocycloaryl and -S heterocycloaryl, wherein the alkyl moieties may be optionally substituted with one or more of the following groups: -SO₂, -SO₂N(C₀₋₁₀ alkyl)(C₀₋₁₀ alkyl), -N(C₀₋₁₀ alkyl)SO₂(C₀₋₁₀ alkyl), -CON(C₀₋₁₀ alkyl)(C₀₋₁₀ alkyl), -N(C₀₋₁₀ alkyl)CO(C₀₋₁₀ alkyl), -N(C₀₋₁₀ alkyl)COO(C₀₋₁₀ alkyl), -OCON(C₀₋₁₀ alkyl)(C₀₋₁₀ alkyl), halogen, -CN, -OCH₂F, -OCHF₂, -OCF₃, -N(C₀₋₁₀ alkyl)(C₀₋₁₀ alkyl), -OC₀₋₁₀ alkyl, -N heterocycloaryl, -O heterocycloaryl and -S heterocycloaryl.

Even more specifically, R₈ and R₉ described above are independently selected from: -H, -CF₃, -CHF₂, -CH₂F, C₁₋₅ linear/branched alkyl, -CH=CH(C₀₋₁₀ alkyl), C₃₋₁₀ cycloalkyl and aromatic six-membered cyclic group, or R₈ and R₉, together with carbon atoms between R₈ and R₉, form C₃₋₆ cycloalkyl, C₄₋₆ fused cycloalkyl, C₅₋₈ spiro cycloalkyl, C₄₋₈ bridged cycloalkyl, C₃₋₇ cyclolactam, C₃₋₇ cyclic lactone, or C₃₋₇ cyclic ketone, wherein H on the C atom may be substituted with the following groups: -SO₂, - SO₂N(C₀₋₁₀ alkyl)(C₀₋₁₀ alkyl), -N(C₀₋₁₀ alkyl)SO₂(C₀₋₁₀ alkyl), -CON(C₀₋₁₀ alkyl)(C₀₋₁₀ alkyl), -N(C₀₋₁₀ alkyl)CO(C₀₋₁₀ alkyl), -N(C₀₋₁₀ alkyl)COO(C₀₋₁₀ alkyl), -OCON(C₀₋₁₀ alkyl)(C₀₋₁₀ alkyl), halogen, - CN, -OCH₂F, -OCHF₂, -OCF₃, C₁₋₁₀ linear/branched alkyl, -N(C₀₋₁₀ alkyl)(C₀₋₁₀ alkyl), -OC₀₋₁₀ alkyl, C₃₋₁₀ cycloalkyl, -O heterocycloalkyl, -N heterocycloalkyl, -N heterocycloaryl, -O heterocycloaryl and -S heterocycloaryl, wherein the alkyl moieties may be optionally substituted with one or more of the following groups: -SO₂, -SO₂N(C₀₋₁₀ alkyl)(C₀₋₁₀ alkyl), -N(C₀₋₁₀ alkyl)SO₂(C₀₋₁₀ alkyl), -CON(C₀₋₁₀ alkyl)(C₀₋₁₀ alkyl), -N(C₀₋₁₀ alkyl)CO(C₀₋₁₀ alkyl), -N(C₀₋₁₀ alkyl)COO(C₀₋₁₀ alkyl), -OCON(C₀₋₁₀ alkyl)(C₀₋₁₀ alkyl), halogen, -CN, -OCH₂F, -OCHF₂, -OCF₃, -N(C₀₋₁₀ alkyl)(C₀₋₁₀ alkyl), -OC₀₋₁₀ alkyl, -N heterocycloaryl, -O heterocycloaryl and -S heterocycloaryl.

In one embodiment of the present invention, R₈ and R₉ described above are independently selected from: -H, -CF₃, -CHF₂, -CH₂F, -CH₃, -CH₂CH₃, -CH=CH₂, 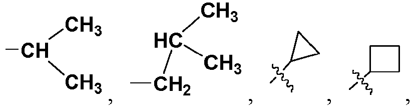 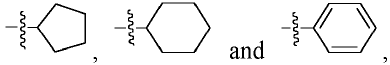 or R₈ and R₉, together with carbon atoms between R₈ and R₉, form

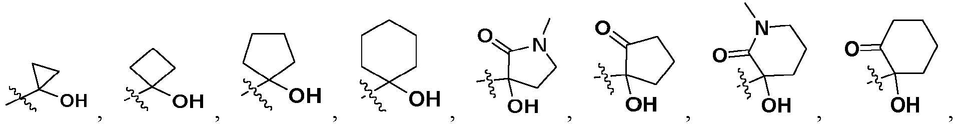

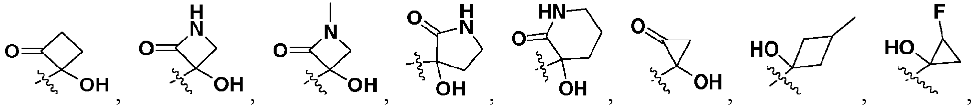

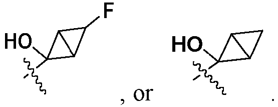

In one embodiment of the present invention, the compound described above has the following structure:

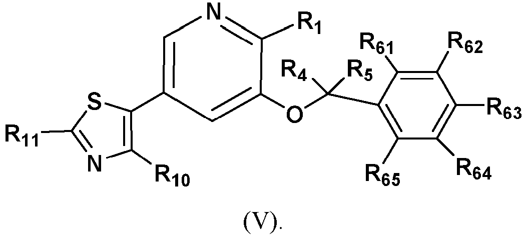

Specifically, the compound described above in the present invention may have the following structures:

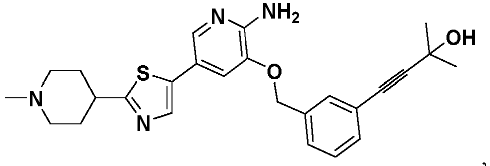

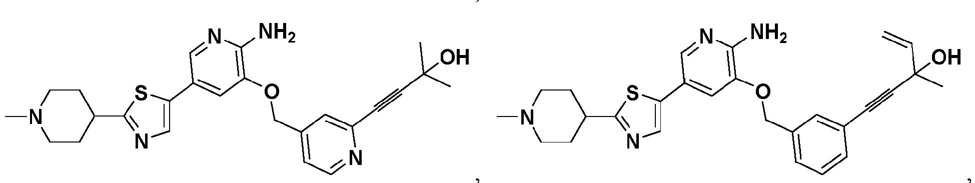

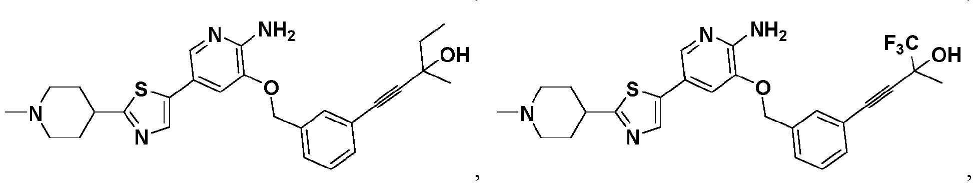

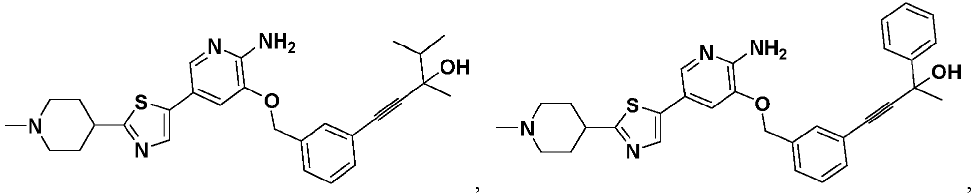

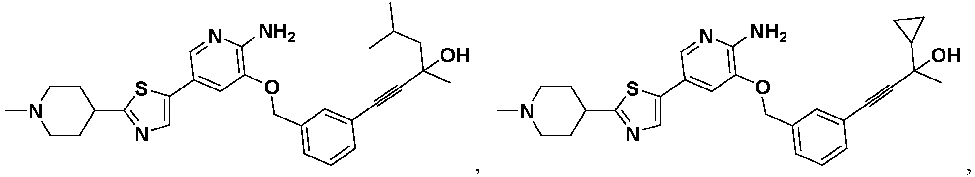

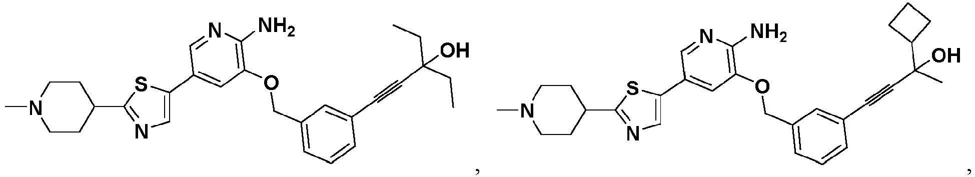

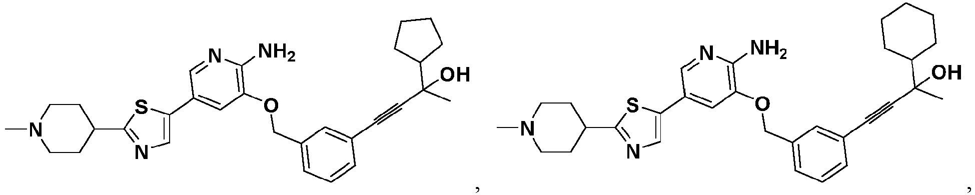

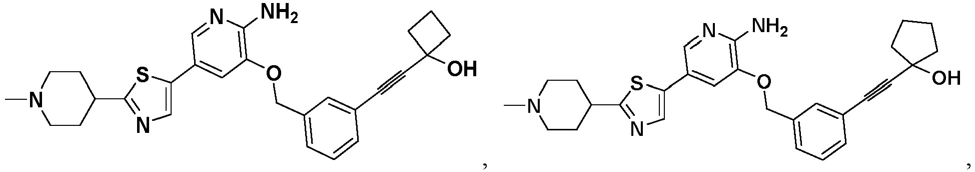

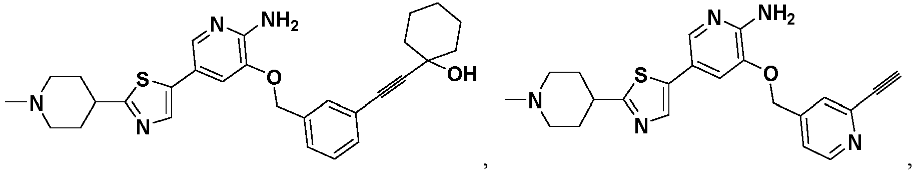

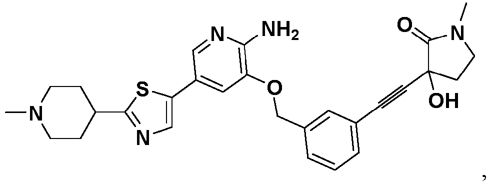

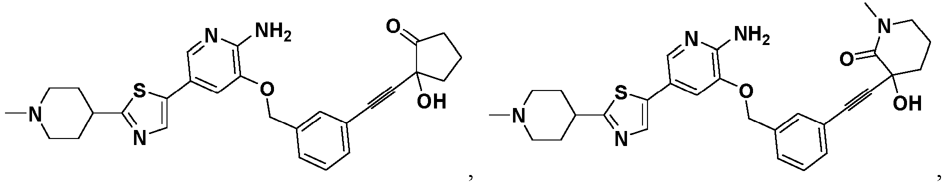

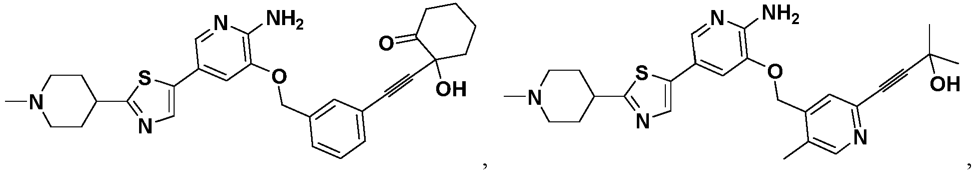

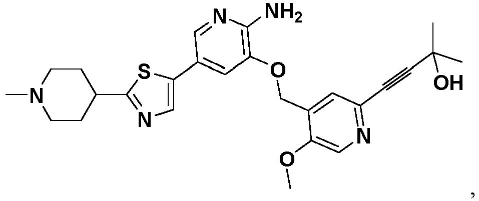

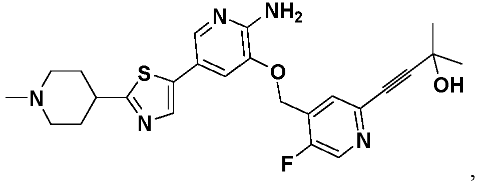

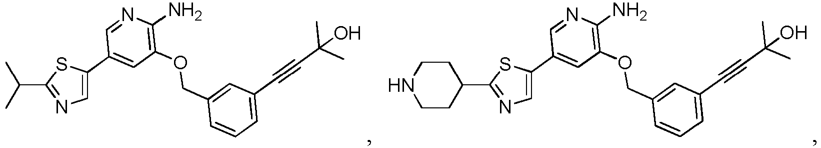

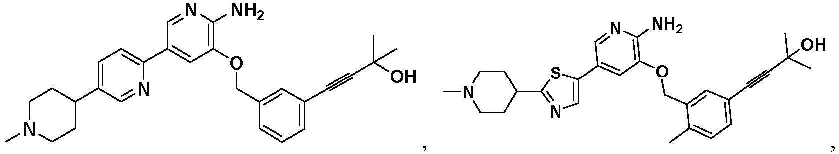

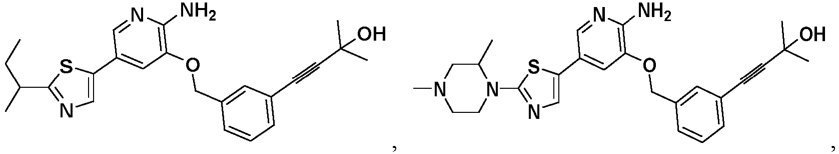

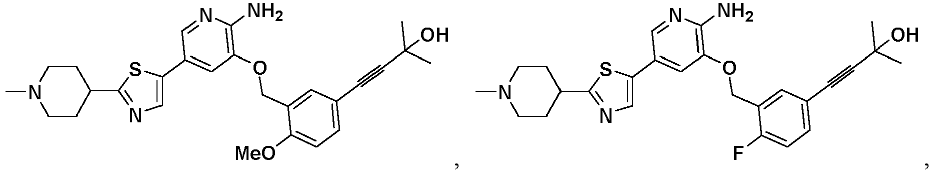

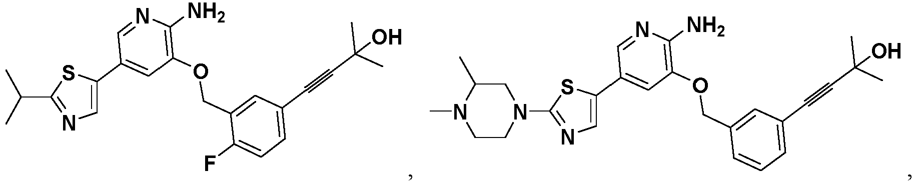

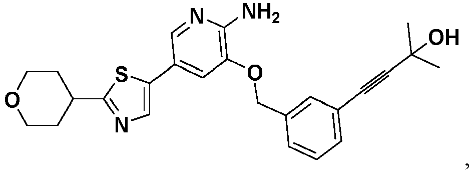

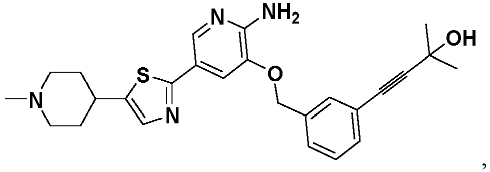

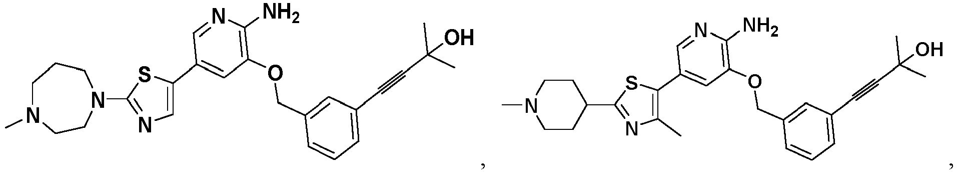

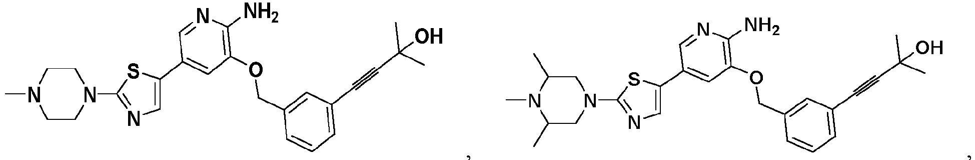

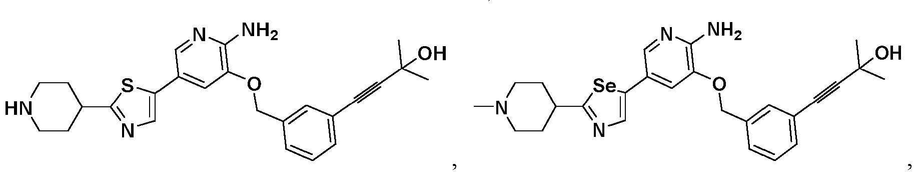

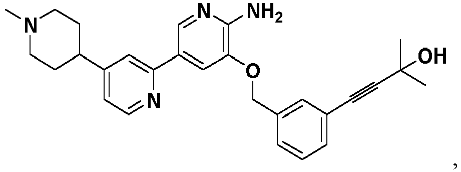

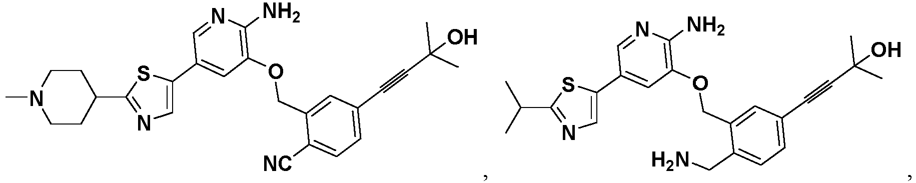

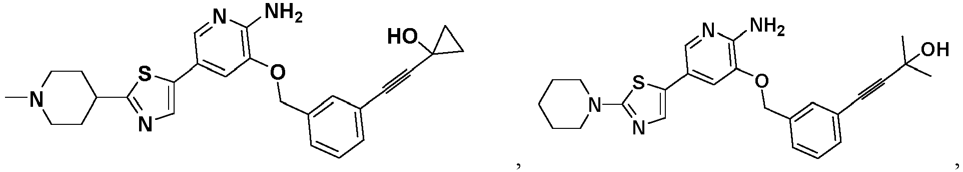

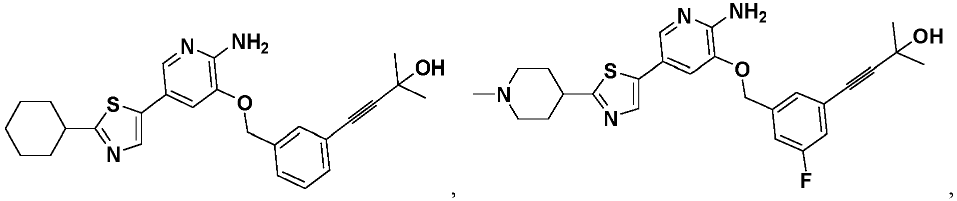

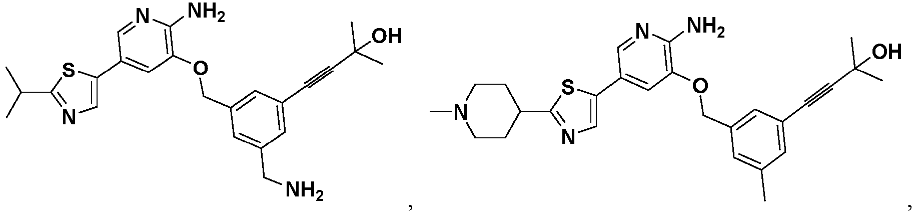

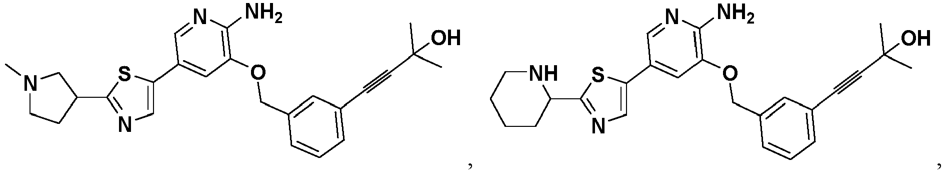

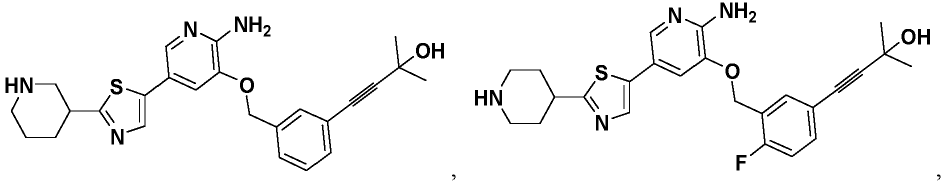

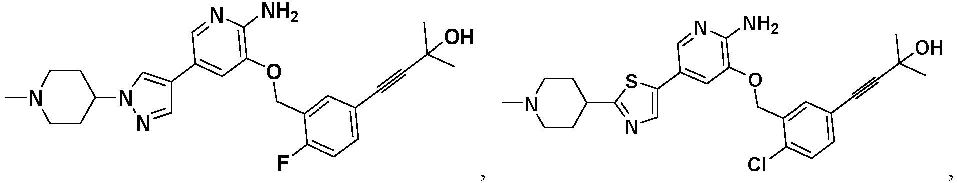

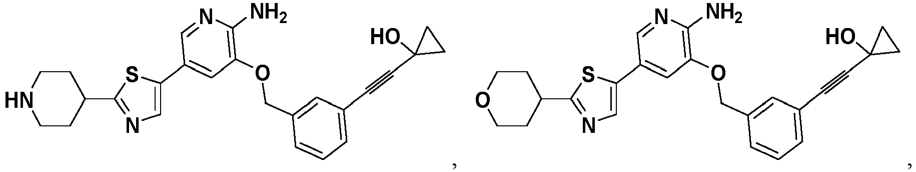

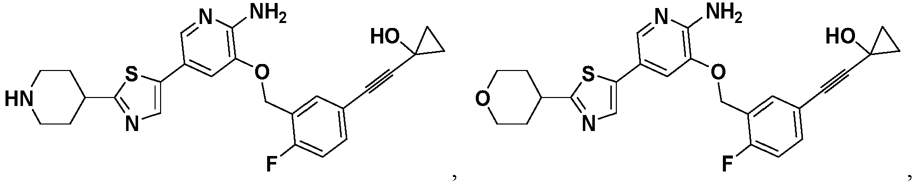

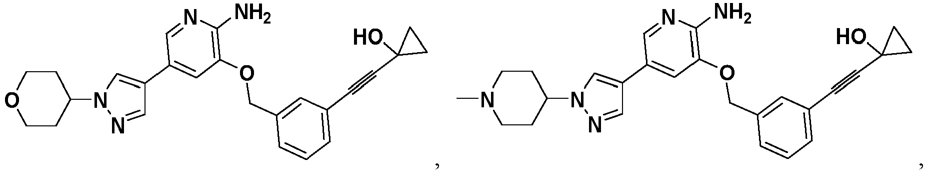

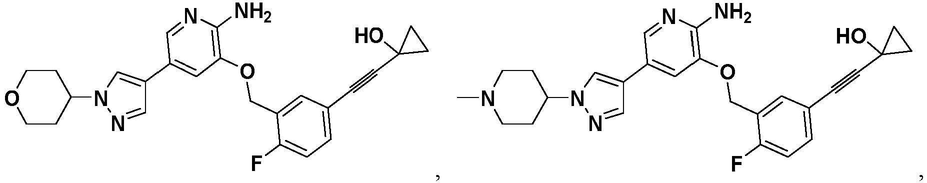

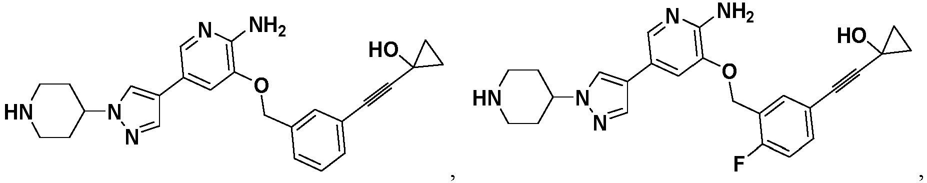

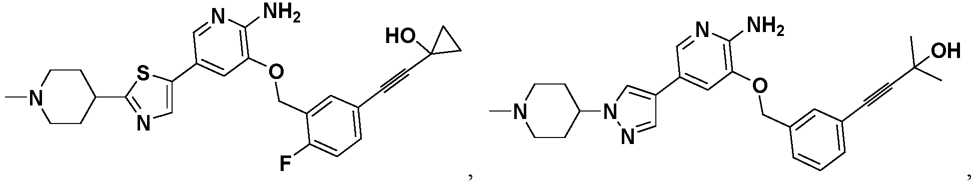

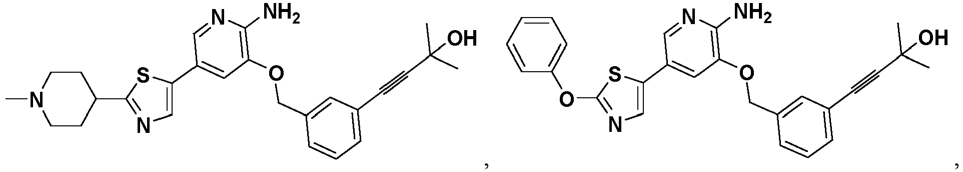

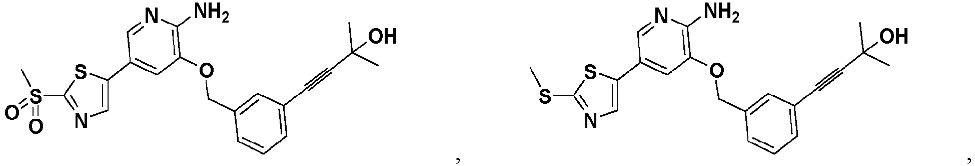

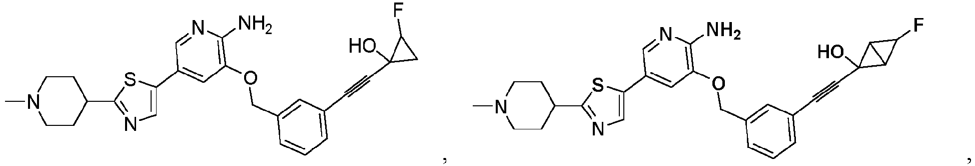

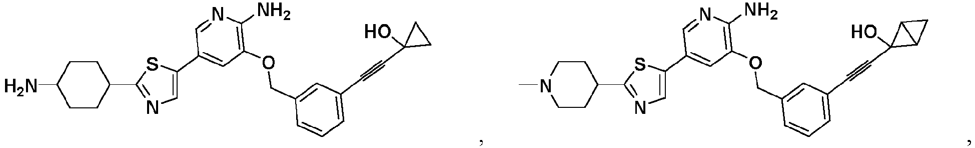

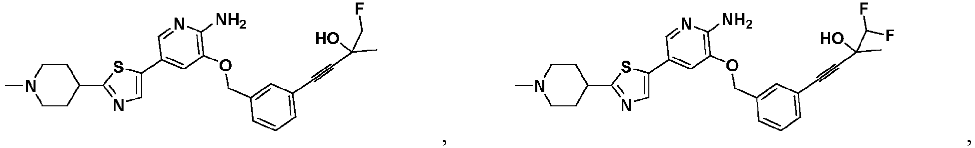

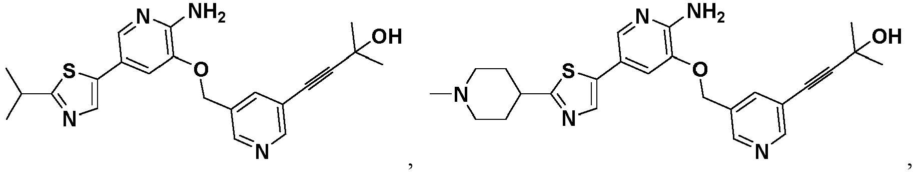

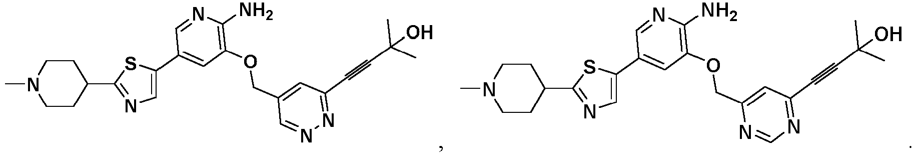

In one embodiment of the present invention, the compound described above has the following structure:

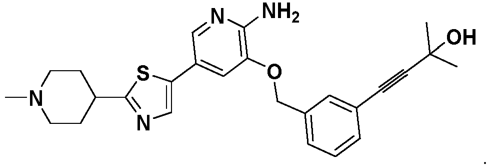

The pharmaceutically acceptable salts of the present invention include acid addition salts and base addition salts.

Specifically, the acid addition salts described above include, but are not limited to, salts derived from inorganic acids, such as hydrochloric acid, nitric acid, phosphoric acid, sulfuric acid, hydrobromic acid, hydroiodic acid and phosphonic acid, and salts derived from organic acids, such as aliphatic mono-carboxylic acid and aliphatic dicarboxylic acid, phenyl-substituted alkanoic acid, hydroxyalkanoic acid, alkanedioic acid, aromatic acid, aliphatic sulfonic acid and aromatic sulfonic acid. Thus, these salts include, but are not limited to, sulfate, pyrosulfate, bisulfate, sulfite, bisulfite, nitrate, phosphate, monohydrogen phosphate, dihydrogen phosphate, metaphosphate, pyrophosphate, hydrochloride, hydrobromide, iodate, acetate, propionate, caprylate, isobutyrate, oxalate, malonate, succinate, suberate, sebacate, fumarate, maleate, mandelate, benzoate, chlorobenzoate, methylbenzoate, dinitrobenzoate, phthalate, benzenesulfonate, tosylate, phenylacetate, citrate, lactate, tartrate, and methanesulfonate, as well as salts comprising amino acids such as arginate, gluconate and galacturonate. The acid addition salts described above can be prepared by contacting the free base form with a sufficient amount of the desired acid to form the salt in a conventional manner. The free base form can be regenerated by contacting the salt form with a base and isolating the free base in a conventional manner.

Specifically, the base addition salts described above are formed with metals or amines, such as hydroxides of alkali metals and alkaline earth metals, or with organic amines. Examples of metals used as cations include, but are not limited to, sodium, potassium, magnesium, and calcium. Examples of suitable amines include, but are not limited to, *N*,*N'*-dibenzylethylenediamine, chloroprocaine, choline, diethanolamine, ethylenediamine(ethane-1,2-diamine), *N*-methylglucamine and procaine. Base addition salts can be prepared by contacting the free acid form with a sufficient amount of the desired base to form the salt in a conventional manner. The free acid form can be regenerated by contacting the salt form with an acid and isolating the free acid in a conventional manner.

Specifically, the solvate described above in the present invention refers to a physical association of the compound of the present invention with one or more solvent molecules. The physical association includes various degrees of ionic and covalent bonding, including hydrogen bonding. In some cases, the solvate can be isolated, for example, when one or more solvent molecules are incorporated into the crystal lattice of a crystalline solid. The "solvate" includes both solution phases and isolatable solvates. Representative solvates include ethanolates, methanolates, and the like. The "hydrate" is a solvate in which one or more solvent molecules are H₂O.

In one embodiment of the present invention, the virus described above is, specifically, for example, avian infectious bronchitis virus (IBV), porcine transmissible gastroenteritis virus (TGEV), porcine epidemic diarrhea virus (PEDV), porcine hemagglutinating encephalomyelitis virus (HEV), mouse hepatitis virus (MHV), turkey bluecomb virus (TCDV), bovine coronavirus (BCV), canine coronavirus (CCV), feline infectious peritonitis virus (FIPV), rat coronavirus (RCV), rat sialodacryoadenitis coronavirus (SDAV), mink epidemic diarrhea coronavirus and the like.

In one embodiment of the present invention, the virus described above is feline infectious peritonitis virus.

As used herein, the term "animal" refers to a non-human animal, particularly to a vertebrate, and specifically to, e.g., a mammal (for example, pig, cattle, sheep, horse, donkey, dog, cat, rabbit, rodent, fox, racoon dog, mink, camel), fish, bird (for example, chicken, duck, goose, pigeon, quail, parrot, etc.), amphibian, reptile, etc., unless otherwise indicated. Particularly, the animal described above is a domestic animal, i.e., an animal raised and domesticated by humans with artificially controlled reproduction for purposes such as food, labor, fur, companionship, experiments, etc., such as an economic animal, a companion animal and a laboratory animal.

In one embodiment of the present invention, in the use described above, the animal is an economic animal, such as livestock (e.g., pig, cattle, sheep, horse, donkey, fox, racoon dog, mink, camel, etc.), poultry (chicken, duck, goose, pigeon, quail, etc.).

In one embodiment of the present invention, in the use described above, the animal is a companion animal, such as dog, cat, rabbit, rodent (e.g., guinea pig, hamster, gerbil, chinchilla, squirrel, etc.), fish, pigeon, parrot, etc.

In one embodiment of the present invention, in the use described above, the animal is a laboratory animal, such as monkey, dog, rabbit, cat, rodent, etc.

In one embodiment of the present invention, the disease described above is selected from avian infectious bronchitis, porcine transmissible gastroenteritis, porcine epidemic diarrhea, canine coronavirus disease, porcine hemagglutinating encephalomyelitis, hepatitis, encephalitis and enteritis caused by mouse hepatitis virus, turkey bluecomb, neonatal calf diarrhea, bovine blood dysentery, feline infectious peritonitis, rat sialodacryoadenitis and mink epidemic diarrhea.

In one embodiment of the present invention, the disease described above is feline infectious peritonitis. Specifically, in the medicament described above, the HPK1 kinase inhibitor described above may be used as the sole active ingredient or may be used in combination with one or more additional active ingredients for the same indication or different indications, wherein the HPK1 kinase inhibitor described above and the additional active ingredients may be formulated for simultaneous, separate or sequential administration.

Specifically, the medicament described above may be in any dosage form or administration form, which can be selected by those skilled in the art according to concrete circumstances. For example, the administration form can be, but is not limited to, oral, sublingual, inhalational, subcutaneous, intramuscular, intravenous, intraperitoneal, intra-organ, intranasal, intrarectal, transdermal, ocular or rectal form; the dosage form can be, but is not limited to, tablets, pills, powders, granules, capsules, lozenges, syrups, solutions, emulsions, suspensions, controlled release formulations, aerosols, films, injections, intravenous drip infusions, transdermal absorption formulations, ointments, lotions, adhesive formulations, suppositories, nasal formulations, pulmonary formulations, eye drops and the like.

In one embodiment of the present invention, the medicament described above is an injection.

The present disclosure further provides use of the HPK1 kinase inhibitor described above in the preparation of a product for resisting pathogen infection in an animal.

Specifically, in the use described above, the HPK1 kinase inhibitor, the pathogen and the animal have the corresponding definitions described above in the present invention.

Specifically, the product described above may be used for either therapeutic purposes or non-therapeutic purposes.

In one embodiment of the present invention, the product described above is a pharmaceutical composition.

Specifically, in the pharmaceutical composition described above, the HPK1 kinase inhibitor described above may be used as the sole active ingredient or may be used in combination with one or more additional active ingredients for the same indication or different indications, wherein the HPK1 kinase inhibitor described above and the additional active ingredients may be formulated for simultaneous, separate or sequential administration.

Specifically, the pharmaceutical composition described above further comprises a pharmaceutically acceptable auxiliary material, in particular an auxiliary material acceptable in the field of veterinary. Specifically, the pharmaceutical composition described above may be in any dosage form or administration form, which can be selected by those skilled in the art according to concrete circumstances. For example, the administration form can be, but is not limited to, oral, sublingual, inhalational, subcutaneous, intramuscular, intravenous, intraperitoneal, intra-organ, intranasal, intrarectal, transdermal, ocular or rectal form; the dosage form can be, but is not limited to, tablets, pills, powders, granules, capsules, lozenges, syrups, solutions, emulsions, suspensions, controlled release formulations, aerosols, films, injections, intravenous drip infusions, transdermal absorption formulations, ointments, lotions, adhesive formulations, suppositories, nasal formulations, pulmonary formulations, eye drops and the like.

In one embodiment of the present invention, the pharmaceutical composition described above is an injection.

The various forms of the pharmaceutical composition described above can be prepared according to conventional production methods in the field of veterinary drugs.

In one embodiment of the present invention, the product described above is a functional food composition.

Specifically, in the functional food composition described above, the compound of formula I may be used as the sole active ingredient, or may be used in combination with one or more additional active ingredients.

Specifically, the functional food composition described above may further comprise an animal food auxiliary material.

Specifically, the functional food composition described above may be in any form, such as tablets, pills, capsules, candies (e.g., tablet candies, gel candies, gum-based candies, etc.), solid beverages (e.g., powders, granules, etc.), liquid beverages and the like.

The various forms of the functional food composition described above can be prepared according to conventional production methods in the field of veterinary functional food.

In one embodiment of the present invention, the product described above is an additive, which can be added in a small amount or in a trace amount during the production, processing and use of the animal food (e.g., livestock and poultry feed, pet daily feed, pet snack, etc.).

The inventor of the present invention found through experiments that the HPK1 kinase inhibitor (especially the compound of formula I, such as 4-(3-{2-amino-5-[2-(1-methyl-piperidin-4-yl)thiazol-5-yl]pyridin-3-yloxymethyl}phenyl)-2-methyl-but-3-yn-2-ol) can effectively treat diseases (such as feline infectious peritonitis) caused by pathogens (specifically viruses, such as coronaviruses) infection in animals, improve animal survival, and provide enhanced commercial value and application prospects in the field of animal antiviral (specifically viruses, such as coronaviruses) medicaments.

### BRIEF DESCRIPTION OF THE DRAWINGS

FIG. 1 illustrates chest X-ray images of an affected cat in Example 1 of the present invention.
FIG. 2 illustrates the clinical survival curve in Example 2 of the present invention, wherein the day when administration started was taken as the first day.

### DETAILED DESCRIPTION

Unless otherwise defined, all scientific and technical terms used herein have the same meaning as commonly understood by those skilled in the art to which the present invention relates.

In the present invention, the term "alkyl" refers to a linear or branched hydrocarbon chain radical without unsaturated bonds, and that is attached to the rest of the molecule via a single bond. Alkyl may be substituted, for example, if alkyl is substituted with cycloalkyl, it is correspondingly "cycloalkylalkyl", such as cyclopropylmethyl; if alkyl is substituted with halogen, it is correspondingly "haloalkyl"; if alkyl is substituted with aryl, it is correspondingly "aralkyl", such as benzyl, benzhydryl or phenethyl; if alkyl is substituted with heterocyclyl, then it is correspondingly "heterocyclylalkyl"; and so on. The C₀₋₁₀ alkyl in the present invention refers to alkyl containing 0 to 10 (e.g., 0, 1, 2, 3, 4, 5, 6, 7, 8, 9 or 10) carbon atoms, such as methyl, ethyl, *n*-propyl, isopropyl, *n*-butyl, isobutyl, *tert-*butyl, *n*-pentyl, isopentyl, neopentyl, *tert*-pentyl, *n*-hexyl, isohexyl, etc., wherein C₀ alkyl refers to H. The term "cycloalkyl" refers to an alicyclic hydrocarbon, for example, containing 1 to 4 monocyclic and/or fused rings and 3 to 18 carbon atoms, preferably 3 to 10 carbon atoms (such as cyclopropyl, cyclohexyl or adamantyl), and the C₃₋₁₀ cycloalkyl in the present invention refers to cycloalkyl containing 3 to 10 (e.g., 3, 4, 5, 6, 7, 8, 9 or 10) carbon atoms, such as cyclopropyl, cyclobutyl, cyclopentyl, cyclohexyl, etc.

The term "aryl" refers to a monocyclic or polycyclic radical, including polycyclic radicals containing monoaryl and/or fused aryl groups, for example, containing 1 to 3 monocyclic or fused rings and 6 to 18 (e.g., 6, 8, 10, 12, 14, 16 or 18) carbon ring atoms, such as phenyl, naphthyl, biphenyl, indenyl, etc. The term "heterocyclyl" includes heteroaromatic and heteroalicyclic groups containing 1 to 3 monocyclic and/or fused rings and 3 to 18 (e.g., 3, 4, 5, 6, 7, 8, 9, 10, 11, 12, 13, 14, 15, 16, 17 or 18) ring atoms. Preferred heteroaromatic groups and heteroalicyclic groups contain 5 to about 10 ring atoms. Suitable heteroaryl in the compound of the present invention contains 1, 2 or 3 heteroatoms which may be selected from N, O and S atoms.

The groups described above in the present invention may be substituted with one or more suitable groups at one or more available positions, such as, but not limited to, OR', =O, SR', SOR', SO₂R', OSO₂R', OSO₃R', NO₂, NHR', N(R')₂, =N-R', N(R')COR', N(COR')₂, N(R')SO₂R', N(R')C(=NR')N(R')R', N₃, CN, halogen, COR', COOR', OCOR', OCOOR', OCONHR', OCON(R')₂, CONHR', CON(R')₂, CON(R')OR', CON(R')SO₂R', PO(OR')₂, PO(OR')R', PO(OR')(N(R')R'), C₁-C₁₂ alkyl, C₃-C₁₀ cycloalkyl, C₂-C₁₂ alkenyl, C₂-C₁₂ alkynyl, aryl and heterocyclyl. Each R' group is independently selected from: hydrogen, OH, NO₂, NH₂, SH, CN, halogen, COH, CO alkyl, COOH, C₁-C₁₂ alkyl, C₃-C₁₀ cycloalkyl, C₂-C₁₂ alkenyl, C₂-C₁₂ alkynyl, aryl, and heterocyclyl; wherein these groups themselves may be substituted, and the substituents may be selected from the foregoing list. The term "halogen", "halogenated" or "halo" refers to bromo, chloro, iodo or fluoro.

The term "treating" includes eradicating, removing, reversing, alleviating, altering or controlling a disease or condition after its onset.

The term "preventing" refers to the ability to avoid, minimize, or make difficult the onset or progression of a disease or condition prior to its onset through treatment.

The term "pathogen" refers to a microorganism (including virus, chlamydia, rickettsia, mycoplasma, bacterium, spirochete, fungus, and the like), parasite (protozoon, worm, and the like) or other vectors that can cause infections or diseases in humans or animals and plants. Among these, the bacterium may be, for example, a Gram-positive cocci, an enterobacterium, a vibrio, a pasteurella, a Gram-negative aerobic bacterium, a Gram-negative microaerophilic and anaerobic bacterium, a Gram-positive non-spore-forming bacillus, a Gram-positive spore-forming bacillus and mycobacterium; the fungus can be, for example, a candida, a cryptococcus and an aspergillus. In the present invention, the pathogen generally refers to a microorganism, a parasite or any other vector that can cause infection or diseases in animals. In one embodiment of the present invention, the pathogen is a virus.

The term "viral infection" refers to the process by which a virus invades the body through a variety of pathways and proliferates in susceptible host cells. The infected body may demonstrate different clinical types. The infection can be classified into apparent infection and latent infection according to the presence or absence of symptoms. An infection where the virus proliferates in host cells but no remarkable clinical symptoms are observed due to the small amount or weak toxicity of the invading virus or the strong resistance of the body is referred to as latent infection. Although a latent infection does not demonstrate clinical symptoms, the virus still proliferates *in vivo* and spreads new viruses to others and becomes an important source of infection. Therefore, resisting viral infection is also required for a host with latent infection. An infection where the virus proliferates in host cells and causes remarkable clinical symptoms due to the large amount or strong toxicity of the invading virus or the weak resistance of the body is referred to as latent infection.

As used herein, the term "animal" refers to a non-human animal, particularly to a vertebrate, and specifically to, e.g., a mammal (for example, pig, cattle, sheep, horse, donkey, dog, cat, rabbit, rodent, fox, racoon dog, mink, camel), fish, bird (for example, chicken, duck, goose, pigeon, quail, parrot, etc.), amphibian and reptile, unless otherwise indicated. Particularly, the animal described above is a domestic animal, i.e., an animal raised and domesticated by humans with artificially controlled reproduction for purposes such as food, labor, fur, companionship and experiment, such as an economic animal, a companion animal and a laboratory animal. The term "economic animal" refers to an animal, such as livestock, poultry and the like, that is raised for meat, milk, fur, labor or other economic purposes; the term "livestock" refers to a domestic animal that is raised and utilized by humans for breeding, and is beneficial to agricultural production; the term "poultry" refers to an avian animal that is fed by humans mainly for meat, eggs, feather or other purposes; the term "companion animal" refers to an animal raised for mental purposes (e.g., for appreciation and companion purposes) rather than economic purposes; the term "laboratory animal" refers to an animal raised for scientific application purposes.

The technical schemes of the present invention will be clearly and completely described below with reference to the examples of the present invention.

### Example 1

### 1. Procedures

Lele, a British shorthair cat, 8 months old, female, unneutered, weighing 2.5 kg. The cat had symptoms of elevated body temperature, mental depression, anorexia, and enlarged abdominal circumference in January, 2020. One week later, the cat was admitted. On January 18, the cat was diagnosed with feline infectious peritonitis (FIP) positive. After being treated with GS-441524 for 3 days, the cat was then given furosemide, Synulox^{®}, prednisolone and itraconazole (P.O., q24h, 10 mg/kg) for 1 month. The drugs were discontinued after ascites regressed. Early in April, the disease recurred, the cat showed decreased food intake and listlessness. X-ray examination suggested hydrothorax, and PCR test showed feline coronavirus positive (the CT value was 25.11). The cat was enrolled on April 8, and was treated with the drug of the present invention at 2.0 mg/kg (SC, q24h) on days 1-14 (D1-14). Proper supportive treatments were given at the same time, including adjuvant treatment such as Moringa (a hepatoprotective drug), Lysinphirst (lysine) and Doctor Dolittle^{®} (lactoferrin).

### 2. Preparation and use

The pharmaceutically active compound described above is a pure powder have the following structure. The compound was added to a 0.9% normal saline (50 mL of endotoxin-free water and 0.45 g of NaCl), and the resulting mixture was uniformly mixed and filtered through a filter membrane to give a clarified solution. The diluted compound was stored in a plastic-sealed sterile vial, stored in a refrigerator at 4°C, gently brought to room temperature before injection, and administered using a disposable syringe. The injection ranged across the back, starting from 2 cm posterior to the scapula to the middle of the lumbar spine, half of the distance of the adjacent chest to flank.

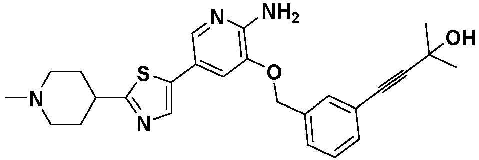

### 3. Results

The treatment was given by injection in the morning every day, body temperature, appetite, mental state and respiratory state were measured and evaluated at 15:00 pm, and urination and defecation conditions were recorded. The results are shown in Table 1.

**Table 1. Experimental results**

| **No.** | **Time** | **Body temperature Morning** | **Body temperature Afternoon** | **Mental state** | **Food intake** | **Respiratory state** | **Defecation condition** | **Notes** |
|---|---|---|---|---|---|---|---|---|
| D0 | 4-8 | | 38.5 | Listless - 3 | Decreased intake - 3 | Normal - 5 | Normal - 5 | 2.5 kg body weight at enrollment |
| D1 | 4-9 | | 38.6 | Slightly improved - 3.5 | Decreased intake - 3 | Normal | Normal | |
| D2 | 4-10 | | 38.1 | Improved -4 | Improved -4 | Normal | Normal | |
| D3 | 4-11 | | 38 | Good - 5 | Good - 5 | Normal | Normal | |
| D4 | 4-12 | | 38.3 | Good | Good | Normal | Normal | |
| D5 | 4-13 | | 38.3 | Good | Good | Normal | Normal | |
| D6 | 4-14 | | 38.3 | Good | Good | Normal | Normal | |
| D7 | 4-15 | | 38.1 | Good | Good | Normal | Normal | 2.75 kg body weight |
| D8 | 4-16 | | 38 | Good | Good | Normal | Normal | |
| D9 | 4-17 | | 38.2 | Good | Good | Normal | Normal | |
| D10 | 4-18 | | 38.3 | Good | Good | Normal | Normal | |
| D11 | 4-19 | | 38.4 | Good | Good | Normal | Normal | |
| D12 | 4-20 | | 38.5 | Good | Good | Normal | Normal | |
| D13 | 4-21 | 38.9 | 38.5 | Good | Good | Normal | Normal | |
| D14 | 4-22 | 38.4 | 38.4 | Good | Good | Normal | Normal | 2.75 kg body weight |

The following procedures were performed on DO, D7 and D14 after enrollment:
1 mL of serum sample was collected and frozen for multi-factor assay. The results are shown in Table 2.

**Table 2. Blood routine test results**

| Blood routine test | | Normal range | D0 | D7 | D14 |
|---|---|---|---|---|---|
| WBC | White blood cell | 5.5~19.5 | 13.5 | 18.7 | 19.7 |
| RBC | Red blood cell | 5.0~10.0 | 10.5 | 7.94 | 7.94 |
| HGB | Hemoglobin | 80~150 | 117 | 94 | 91 |
| HCT | Hematocrit | 24.0~45.0 | 37.5 | 28.9 | 29.9 |
| MCV | Mean corpuscular volume | 39.0~55.0 | 35.9 | 36.4 | 37.7 |
| MCH | Mean corpuscular hemoglobin | 12.5~17.5 | 11.2 | 11.8 | 11.5 |

### 3) Chest X-ray

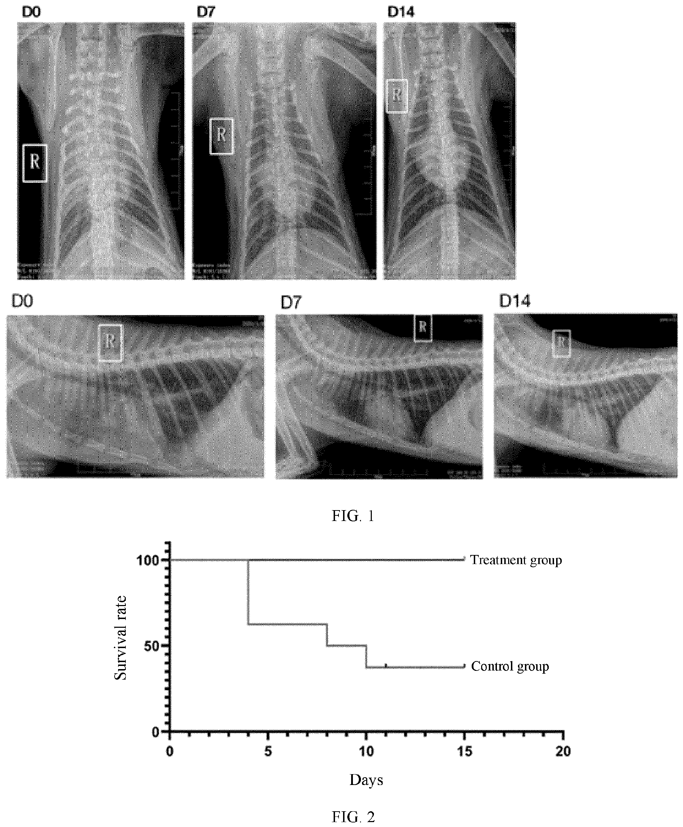

As shown in FIG. 1.

### 4) Analysis of results

The above results suggested wet FIP (in the chest), showing hydrothorax, listlessness and anorexia, which were typical clinical symptoms of feline infectious peritonitis.

Because the cat had no fever symptom at enrollment, the fever alleviation effect cannot be observed during treatment, and the body temperature was always maintained at in the normal range, 38-38.5°C, throughout the treatment. After 2 weeks of treatment, the cat demonstrated remarkable clinical response with improved appetite and mental state, and weight gain. Since about 7 days after the treatment, the hydrothorax completely regressed. The body weight increased by 9% during and after the treatment.

### Example 2

A total of 15 cats with clinically confirmed FIP were enrolled, with hydrothorax/ascites, positive CT value by PCR, and no neurological symptoms. 8 cats were allocated into the control group, and 7 cats were allocated into the treatment group.

The groups were pre-treated with GS-441524 for 3 days. For the control group, necessary supportive treatment was given on D1-D14. For the treatment group, after 3 days of observation, necessary supportive treatment and the drug of the present invention (2.0 mg/kg, SC, q24h) were given on D1-D14.

The occurrence of death or neurological symptoms was judged as clinical death, and the clinical survival curve was plotted after 14 days. The results are shown in FIG. 2. Log Rank (Mantel-Cox) and Breslow (Generalized Wilcoxon) methods were used for validity test, and both methods indicated a significant difference (*P* < *0.05*)*.*

## Claims

1. A compound, or a pharmaceutically acceptable salt, a solvate or a deuterated compound thereof for use in preventing and/or treating a disease caused by or associated with a pathogen infection in an animal;
the compound has the following structure: 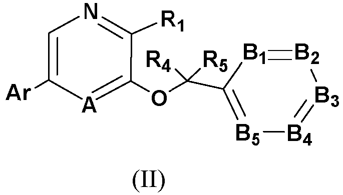 wherein,
A is selected from C and N;
Ar is selected from: 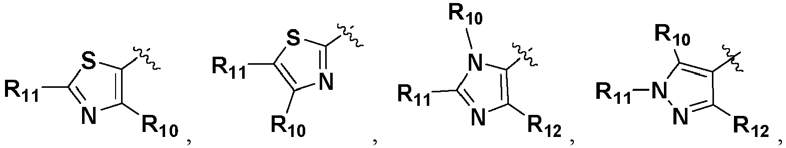 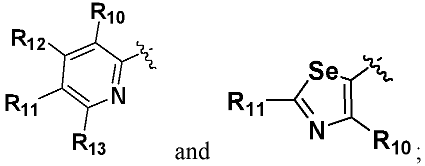
wherein R₁₀, R₁₂ and R₁₃ are independently selected from: -H, C₁₋₁₀ linear/branched alkyl, -N(C₀₋₁₀ alkyl)(C₀₋₁₀ alkyl), -OC₀₋₁₀ alkyl and C₃₋₁₀ cycloalkyl;
R₁₁ is selected from: -H, -O heterocycloalkyl, -N heterocycloalkyl, C₁₋₁₀ linear/branched alkyl, C₃₋₁₀ cycloalkyl, -OC₀₋₁₀ alkyl, -N(C₀₋₁₀ alkyl)(C₀₋₁₀ alkyl), -SO₂(C₀₋₁₀ alkyl), -O(C₀₋₁₀ alkyl), -O-phenyl, - S(C₀₋₁₀ alkyl), -N heterocycloaryl, -O heterocycloaryl and -S heterocycloaryl, wherein H on the C atom or heteroatom may be substituted with C₁₋₃ linear alkyl, -N(C₀₋₁₀ alkyl)(C₀₋₁₀ alkyl) or -CF₃;
R₁ is selected from: -H, halogen, -NO₂, -CN, C₁₋₅ linear/branched alkyl, C₃₋₁₀ cycloalkyl, -N(C₀₋₁₀ alkyl)(C₀₋₁₀ alkyl), -CF₃, -OCF₃, -OCHF₂, -OCH₂F and -OC₀₋₁₀ alkyl;
R₄ and R₅ are independently selected from: -H, deuterium, halogen, C₁₋₁₀ linear/branched alkyl and deuterated C₁₋₁₀ linear/branched alkyl;
at least one of B₁, B₂, B₃, B₄ and B₅ is N; or 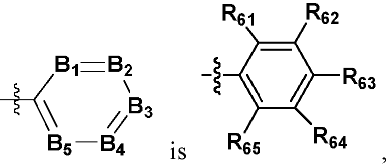 wherein
R₆₃ is selected from: -H, halogen, -OC₀₋₁₀ alkyl, C₁₋₁₀ linear/branched alkyl, -N(C₀₋₁₀ alkyl)(C₀₋₁₀ alkyl) and C₃₋₁₀ cycloalkyl;
R₆₁, R₆₄ and R₆₅ are independently selected from: -H, halogen, -CN, -OC₀₋₁₀ alkyl, C₁₋₁₀ linear/branched alkyl, -N(C₀₋₁₀ alkyl)(C₀₋₁₀ alkyl), C₃₋₁₀ cycloalkyl, -C≡C-R₇, -O heterocycloalkyl, -N heterocycloalkyl and C₁₋₅ linear/branched alkyl containing O and N;
R₆₂ is C≡C-R₇;
R₇ is selected from H, C₁₋₅ linear/branched alkyl, C₃₋₁₀ cycloalkyl and 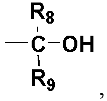 wherein R₈ and R₉ are independently selected from: -H, -CF₃, -CHF₂, -CH₂F, C₁₋₁₀ linear/branched alkyl, -CH=C(C₀₋₁₀ alkyl)(C₀₋₁₀ alkyl), -C≡C(C₀₋₁₀ alkyl), C₃₋₁₀ cycloalkyl, -CO(C₀₋₁₀ alkyl)(C₀₋₁₀ alkyl), -CO(C₀₋₁₀ alkyl)(C₀₋₁₀ alkyl), -CON(C₀₋₁₀ alkyl)(C₀₋₁₀ alkyl), -N(C₀₋₁₀ alkyl)CO(C₀₋₁₀ alkyl)(C₀₋₁₀ alkyl), aromatic five-membered cyclic group and aromatic six-membered cyclic group, or R₈ and R₉, together with carbon atoms between R₈ and R₉, form C₃₋₈ cycloalkyl or C₃₋₈ heterocycloalkyl containing -O- or -S-, C₄₋₉ fused cycloalkyl, C₅₋₁₀ spiro cycloalkyl, C₄₋₉ bridged cycloalkyl, C₃₋₇ cyclolactam, C₃₋₇ cyclic lactone or C₃₋₇ cyclic ketone; and
the pathogen is a coronavirus.

2. The compound for the use according to claim 1, wherein R₁₁ is selected from:

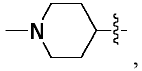

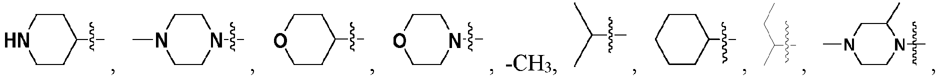

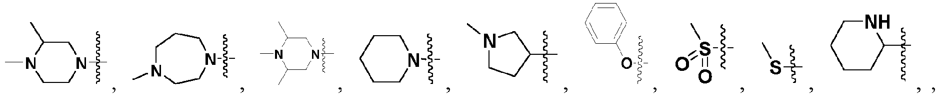

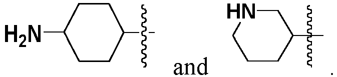

3. The compound for the use according to claim 1, wherein R₆₃ is selected from: -H, halogen, -OC₀₋₁₀ alkyl and C₁₋₁₀ linear/branched alkyl.

4. The compound for the use according to claim 1, wherein R₆₃ is selected from: -H, -F and -OCH₃.

5. The compound for the use according to claim 1, wherein R₆₁, R₆₄ and R₆₅ are independently selected from: -H, halogen, C₁₋₃ linear/branched alkyl, -OC₀₋₃ alkyl and C₁₋₃ linear/branched alkyl containing N.

6. The compound for the use according to claim 1, wherein R₆₁, R₆₄ and R₆₅ are independently selected from: -H, -F, -Cl, -CH₃, -CH₂NH₂, -CN and -OCH₃.

7. The compound for the use according to claim 1, wherein R₈ and R₉ are independently selected from: -H, -CF₃, -CHF₂, -CH₂F, -CH₃, -CH₂CH₃, -CH=CH₂, 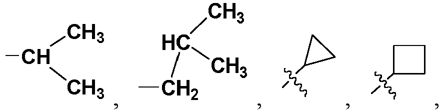 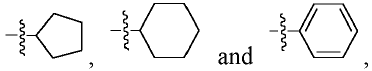 or R₈ and R₉, together with carbon atoms between R₈ and R₉, form

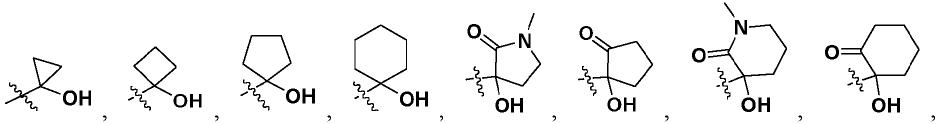

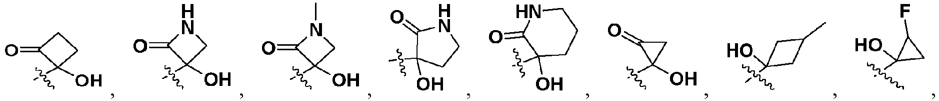

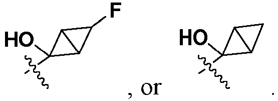

8. The compound for the use according to claim 1, wherein the compound is selected from:

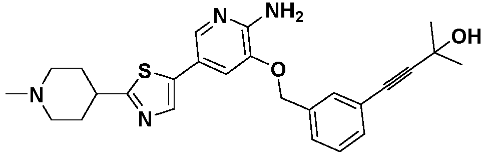

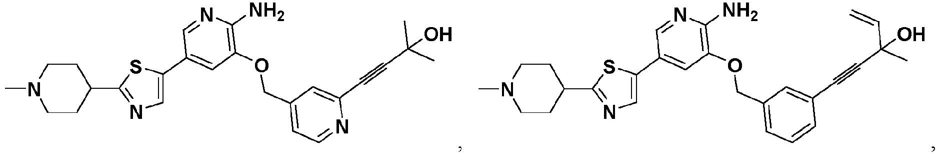

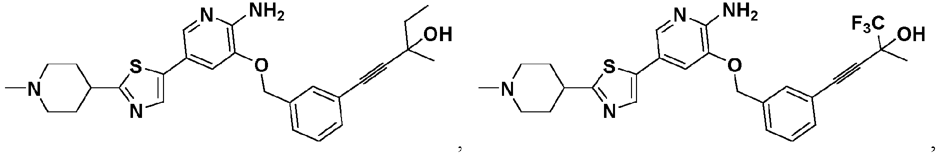

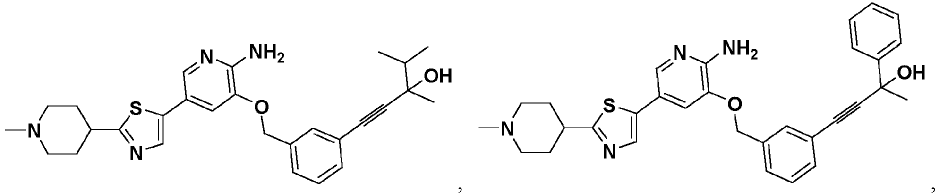

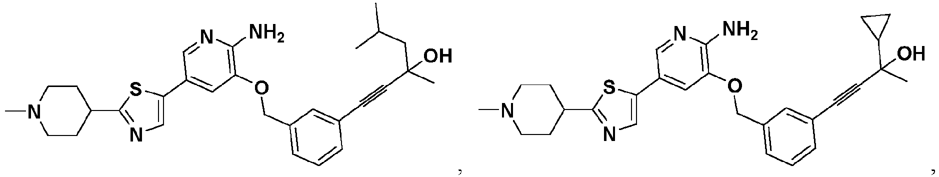

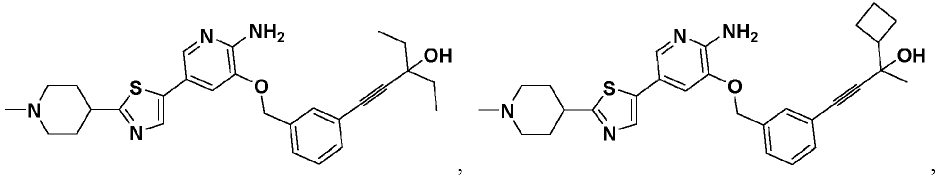

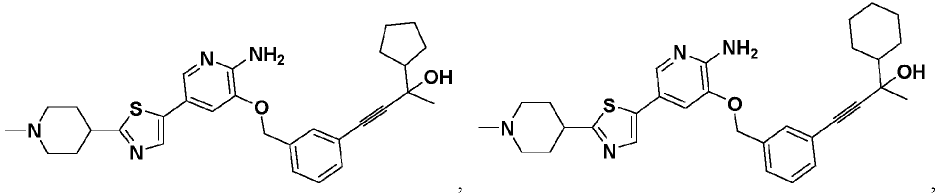

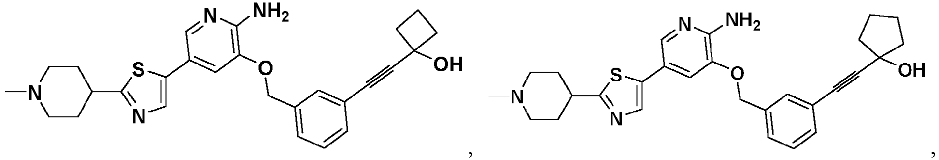

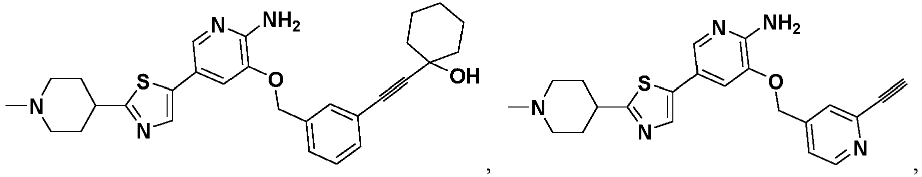

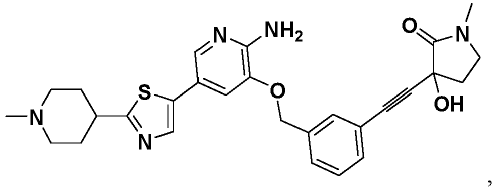

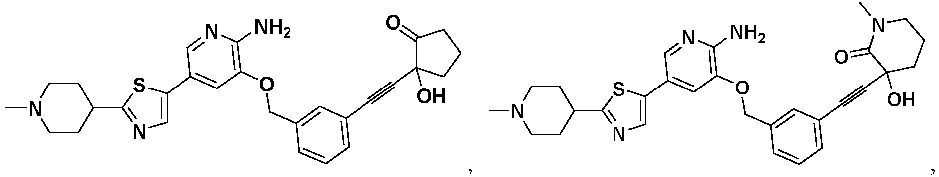

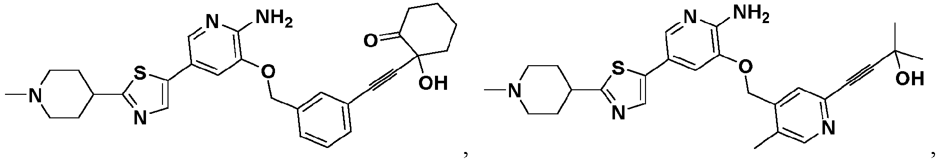

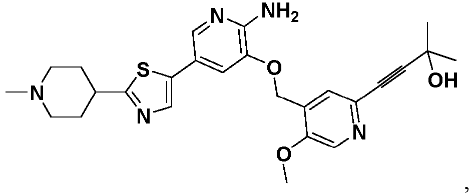

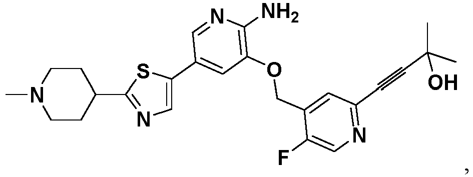

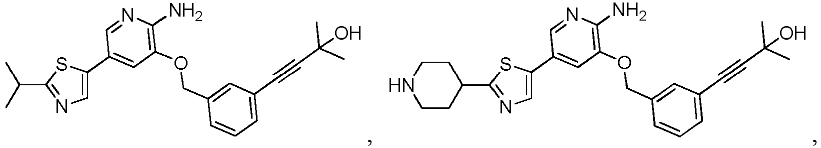

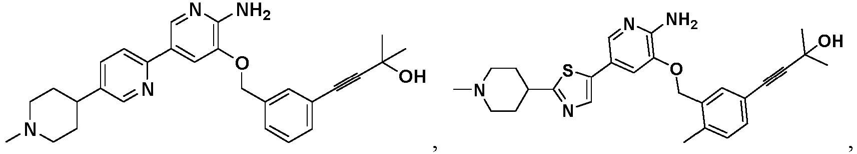

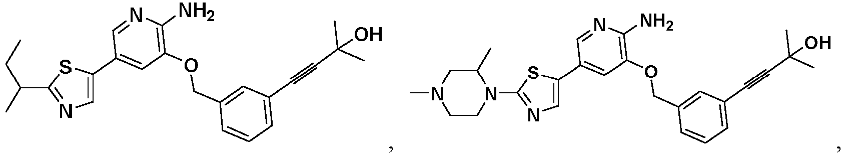

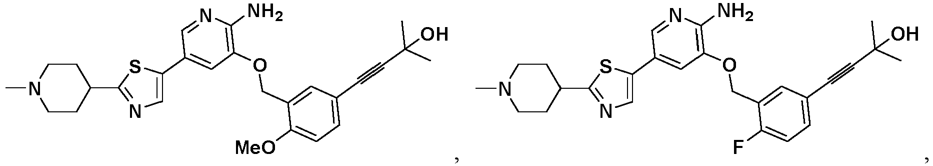

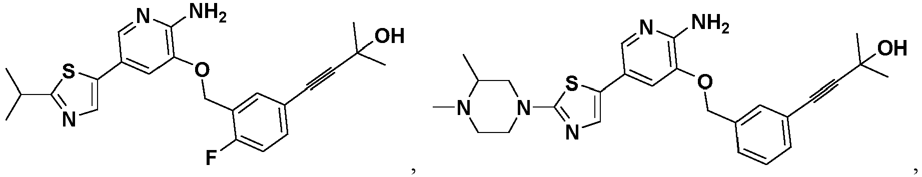

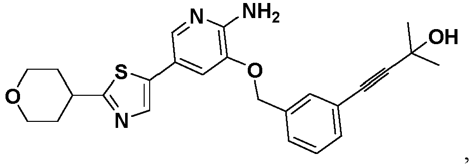

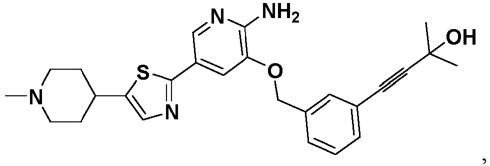

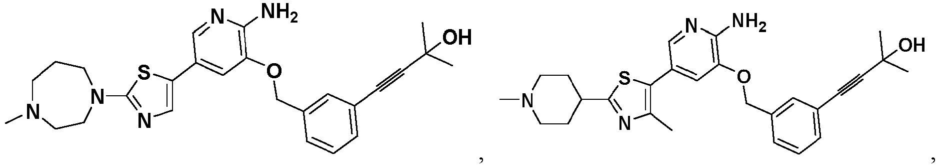

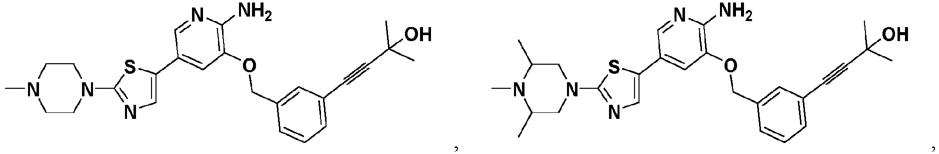

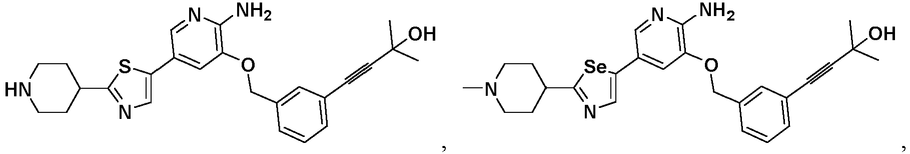

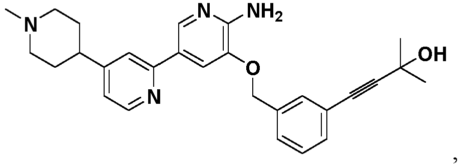

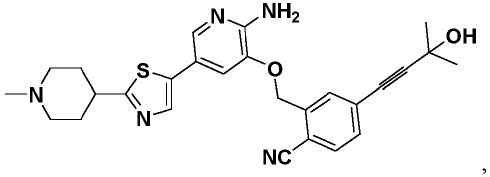

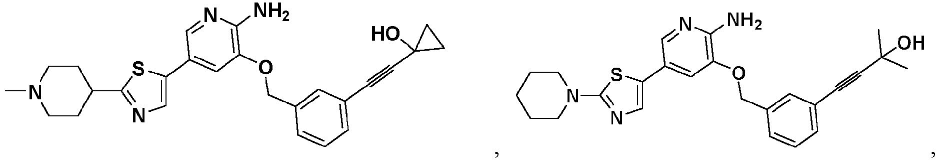

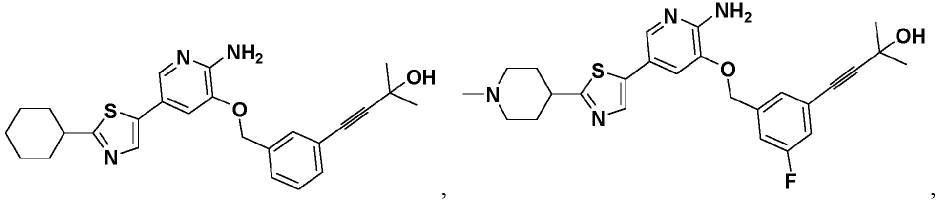

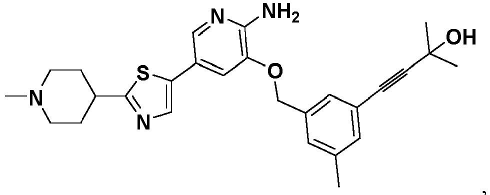

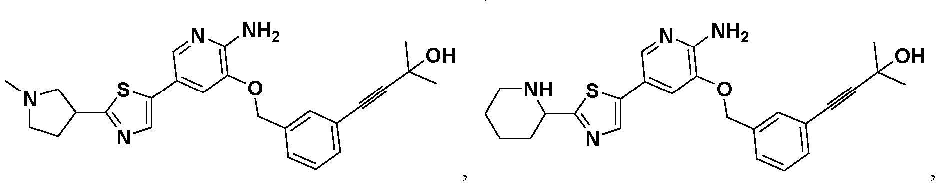

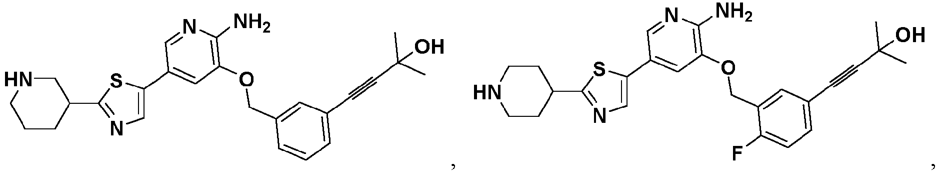

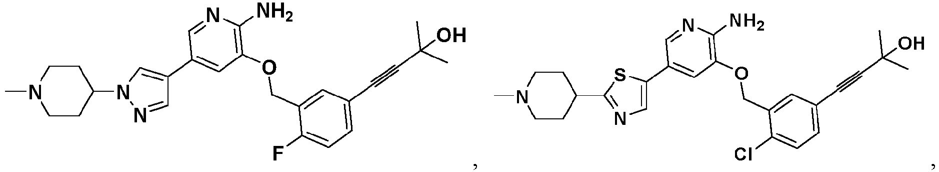

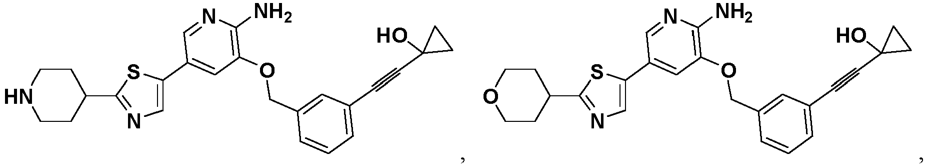

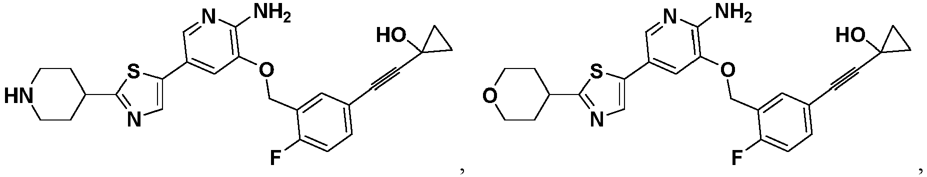

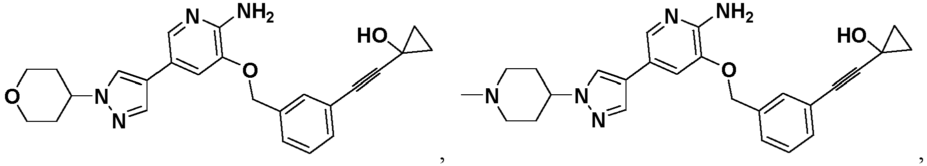

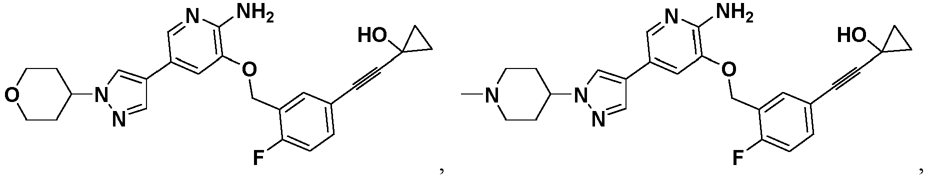

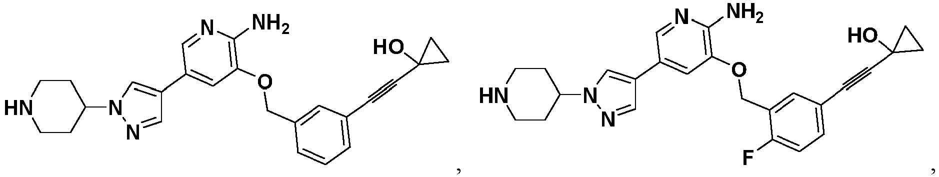

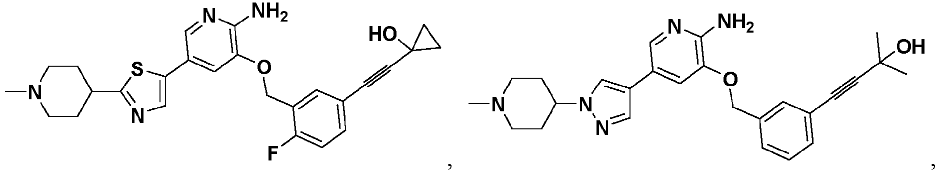

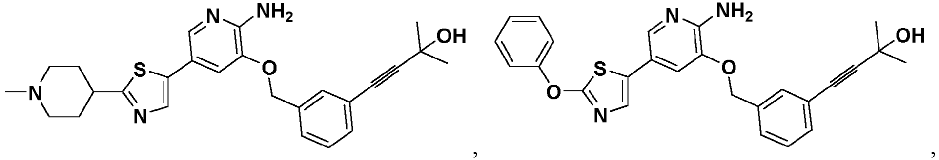

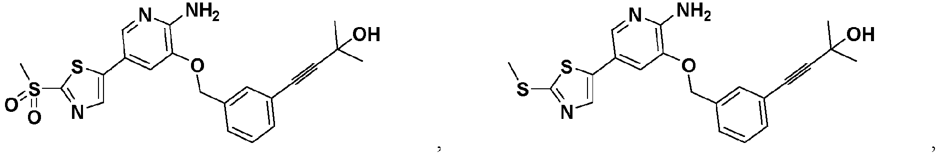

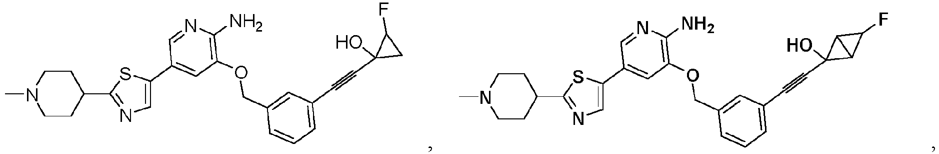

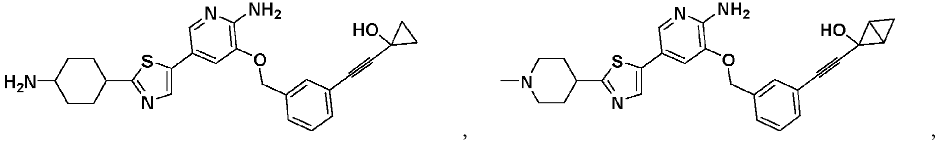

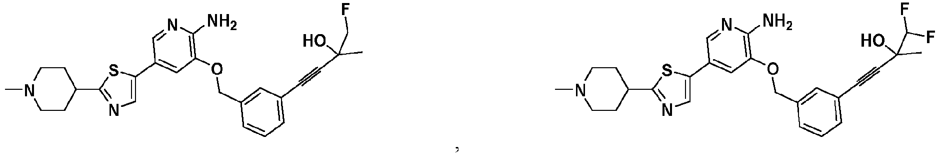

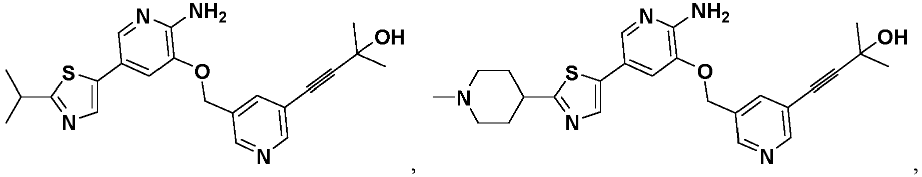

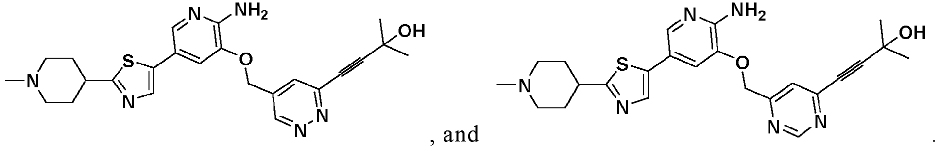

9. The compound for the use according to claim 1, wherein the coronavirus is selected from avian infectious bronchitis virus, porcine transmissible gastroenteritis virus, porcine epidemic diarrhea virus, porcine hemagglutinating encephalomyelitis virus, mouse hepatitis virus, turkey bluecomb virus, bovine coronavirus, canine coronavirus, feline infectious peritonitis virus, rat coronavirus, rat sialodacryoadenitis coronavirus and mink epidemic diarrhea coronavirus.

10. The compound for the use according to claim 1, wherein the coronavirus is feline infectious peritonitis virus.

11. The compound for the use according to claim 1, wherein the animal is a domestic animal.

12. The compound for the use according to claim 1, wherein the animal is a companion animal or a laboratory animal.

13. The compound for the use according to claim 1, wherein the animal is livestock or poultry.

14. The compound for the use according to claim 1, wherein the disease is selected from avian infectious bronchitis, porcine transmissible gastroenteritis, porcine epidemic diarrhea, canine coronavirus disease, porcine hemagglutinating encephalomyelitis, hepatitis, encephalitis and enteritis caused by mouse hepatitis virus, turkey bluecomb, neonatal calf diarrhea, bovine blood dysentery, feline infectious peritonitis, rat sialodacryoadenitis and mink epidemic diarrhea.

15. The compound for the use according to claim 1, wherein the disease is feline infectious peritonitis.

## Patentansprüche

1. Verbindung oder pharmazeutisch verträgliches Salz, Solvat oder deuterierte Verbindung davon zur Verwendung bei der Vorbeugung und/oder Behandlung einer Krankheit, die durch eine pathogene Infektion bei einem Tier verursacht wird oder damit in Zusammenhang steht;
wobei die Verbindung die folgende Struktur aufweist: 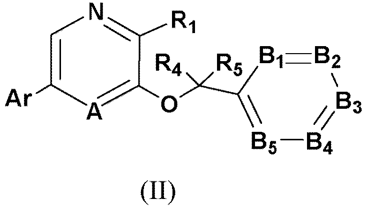 wobei
A aus C und N ausgewählt ist; 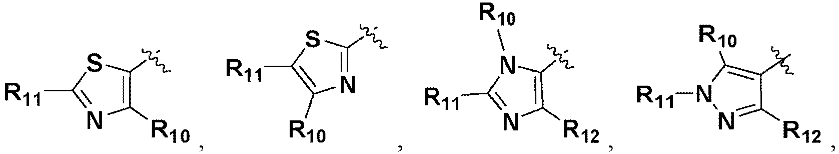
Ar aus Folgendem ausgewählt ist: 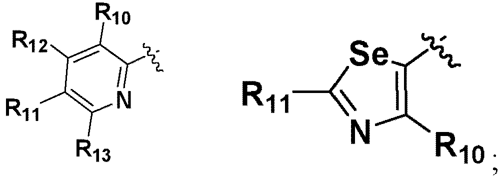 und
wobei R₁₀, R₁₂ und R₁₃ unabhängig voneinander aus Folgendem ausgewählt sind: -H, linearem/verzweigtem C₁₋₁₀-Alkyl, - N(C₀₋₁₀-Alkyl)(C₀₋₁₀-Alkyl), -OC₀₋₁₀-Alkyl und C₃₋₁₀-Cycloalkyl;
R₁₁ aus Folgendem ausgewählt ist: -H, -O-Heterocycloalkyl, -N-Heterocycloalkyl, linearem/verzweigtem C₁₋₁₀-Alkyl, C₃₋₁₀-Cycloalkyl, -OC₀₋₁₀-Alkyl, -N(C₀₋₁₀-Alkyl)(C₀₋₁₀-Alkyl), - SO₂(C₀₋₁₀-Alkyl), -O(C₀₋₁₀-Alkyl), -O-Phenyl, -S(C₀₋₁₀-Alkyl), -N-Heterocycloaryl, -O-Heterocycloaryl und -S-Heterocycloaryl, wobei H am C-Atom oder Heteroatom durch lineares C₁₋₃-Alkyl, -N(C₀₋₁₀-Alkyl)(C₀₋₁₀-Alkyl) oder -CF₃ ersetzt sein kann;
R₁ aus Folgendem ausgewählt ist: -H, Halogen, -NO₂, -CN, linearem/verzweigtem C₁₋₅-Alkyl, C₃₋₁₀-Cycloalkyl, -N(C₀₋₁₀-Alkyl) (C₀₋₁₀-Alkyl), -CF₃, -OCF₃, -OCHF₂, -OCH₂F und -OC₀₋₁₀-Alkyl;
R₄ und R₅ unabhängig voneinander aus Folgendem ausgewählt sind: -H, Deuterium, Halogen, linearem/verzweigtem C₁₋₁₀-Alkyl und linearem/verzweigtem deuteriertem C₁₋₁₀-Alkyl; mindestens eines von B₁, B₂, B₃, B₄ und B₅ N ist; oder 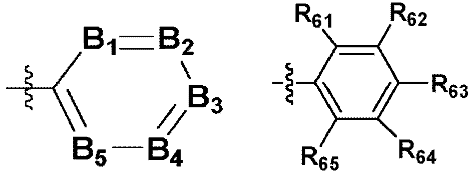 ist, wobei
R₆₃ aus Folgendem ausgewählt ist: -H, Halogen, -OC₀₋₁₀-Alkyl, linearem/verzweigtem C₁₋₁₀-Alkyl, -N(C₀₋₁₀-Alkyl)(C₀₋₁₀-Alkyl) und C₃₋₁₀-Cycloalkyl;
R₆₁, R₆₄ und R₆₅ unabhängig voneinander aus Folgendem ausgewählt sind: -H, Halogen, -CN, -OC₀₋₁₀-Alkyl, linearem/verzweigtem C₁₋₁₀-Alkyl, -N(C₀₋₁₀-Alkyl)(C₀₋₁₀-Alkyl), C₃₋₁₀-Cycloalkyl, -C≡C-R₇, -O-Heterocycloalkyl, -N-Heterocycloalkyl und linearem/verzweigtem C₁₋₅-Alkyl, das O und N enthält;
R₆₂ C≡C-R₇ ist;
R₇ aus H, linearem/verzweigtem C₁₋₅-Alkyl, C₃₋₁₀-Cycloalkyl und 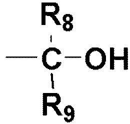 ausgewählt ist, wobei R₈ und R₉ unabhängig voneinander aus Folgendem ausgewählt sind: -H, -CF₃, -CHF₂, -CH₂F, linearem/verzweigtem C₁₋₁₀-Alkyl, -CH=C(C₀₋₁₀-Alkyl)(C₀₋₁₀-Alkyl), -C≡C(C₀₋₁₀-Alkyl), C₃₋₁₀-Cycloalkyl, - CO(C₀₋₁₀-Alkyl)(C₀₋₁₀-Alkyl), -CO(C₀₋₁₀-Alkyl)(C₀₋₁₀-Alkyl), - CON(C₀₋₁₀-Alkyl)(C₀₋₁₀-Alkyl), -N(C₀₋₁₀-Alkyl)CO(C₀₋₁₀-Alkyl) (C₀₋₁₀-Alkyl), einer aromatischen fünfgliedrigen cyclischen Gruppe und einer aromatischen sechsgliedrigen cyclischen Gruppe, oder R₈ und R₉ zusammen mit Kohlenstoffatomen zwischen R₈ und R₉ C₃₋₈-Cycloalkyl oder C₃₋₈-Heterocycloalkyl, das -O- oder -S- enthält, ein kondensiertes C₄₋₉-Cycloalkyl, C₅₋₁₀-Spirocycloalkyl, ein verbrücktes C₄₋₉-Cycloalkyl, C₃₋₇-Cyclolactam, ein cyclisches C₃₋₇-Lacton oder ein cyclisches C₃₋₇-Keton bilden; und
der Erreger ein Coronavirus ist.

2. Verbindung zur Verwendung nach Anspruch 1, wobei R₁₁ aus Folgendem ausgewählt ist:

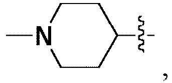

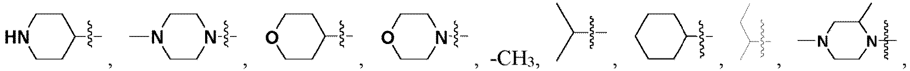

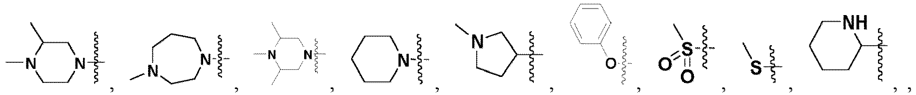

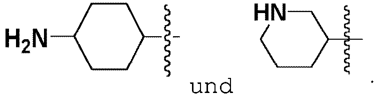

3. Verbindung zur Verwendung nach Anspruch 1, wobei R₆₃ aus Folgendem ausgewählt ist: -H, Halogen, -OC₀₋₁₀-Alkyl und linearem/verzweigtem C₁₋₁₀-Alkyl.

4. Verbindung zur Verwendung nach Anspruch 1, wobei R₆₃ aus Folgendem ausgewählt ist: -H, -F und -OCH₃.

5. Verbindung zur Verwendung nach Anspruch 1, wobei R₆₁, R₆₄ und R₆₅ unabhängig voneinander aus Folgendem ausgewählt sind: -H, Halogen, linearem/verzweigtem C₁₋₃-Alkyl, -OC₀₋₃-Alkyl und linearem/verzweigtem C₁₋₃-Alkyl, das N enthält.

6. Verbindung zur Verwendung nach Anspruch 1, wobei R₆₁, R₆₄ und R₆₅ unabhängig voneinander aus Folgendem ausgewählt sind: -H, -F, -Cl, -CH₃, -CH₂NH₂, -CN und -OCH₃.

7. Verbindung zur Verwendung nach Anspruch 1, wobei R₈ und R₉ unabhängig voneinander aus Folgendem ausgewählt sind: - H, -CF₃, -CHF₂, -CH₂F, -CH₃, -CH₂CH₃, -CH=CH₂, 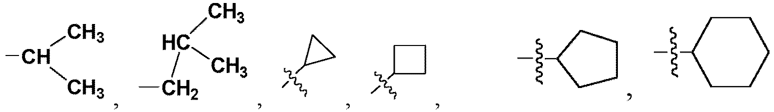 und 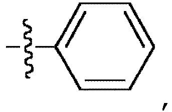 oder R₈ und R₉ zusammen mit Kohlenstoffatomen zwischen R₈ und R₉ 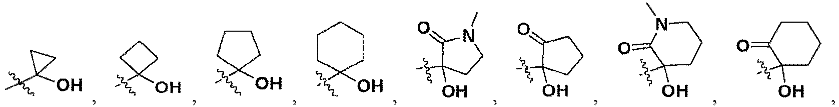 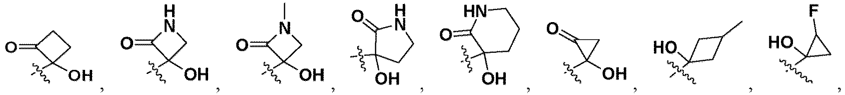 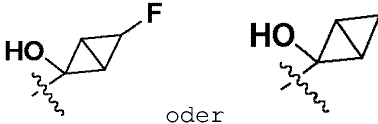 bilden.

8. Verbindung zur Verwendung nach Anspruch 1, wobei die Verbindung aus Folgendem ausgewählt ist: 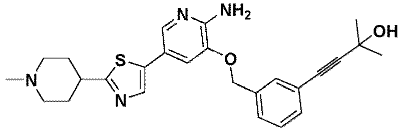 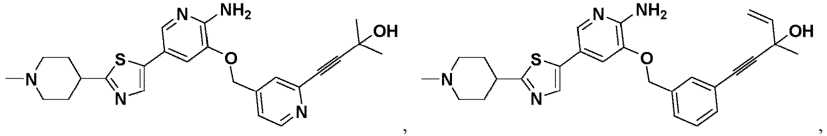 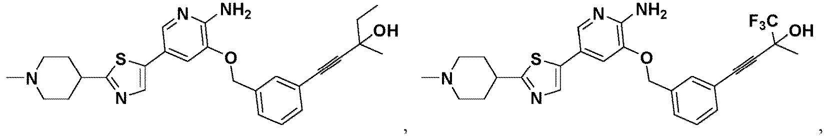 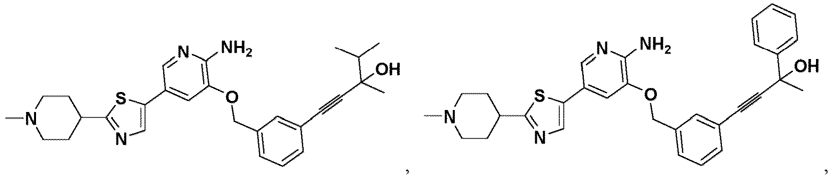 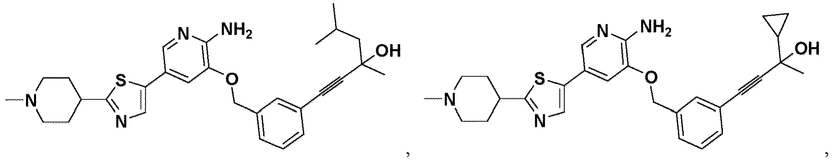 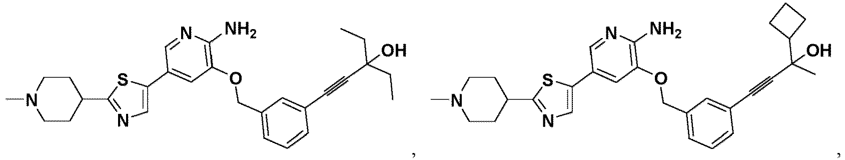 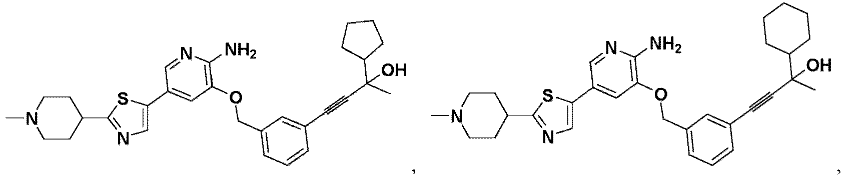 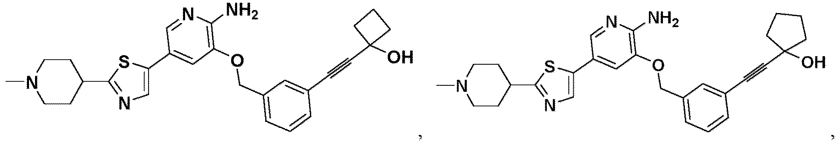 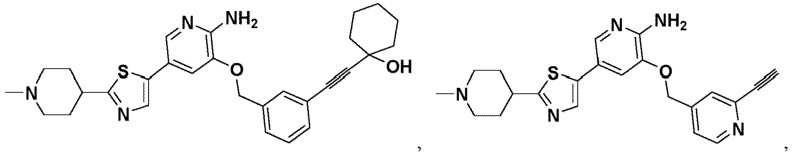 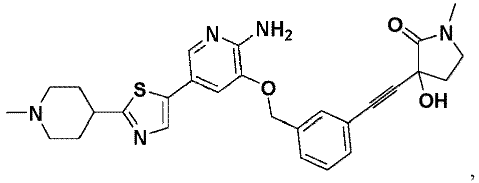 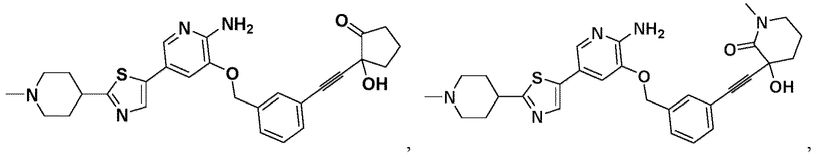 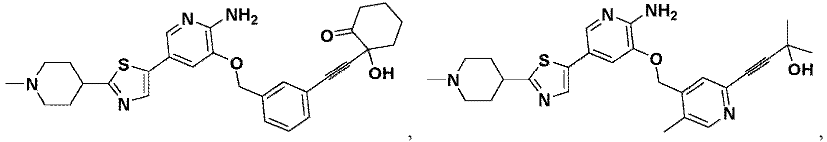 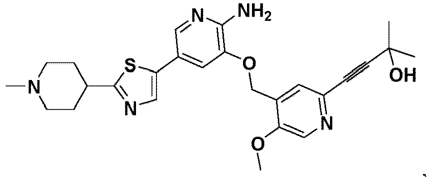 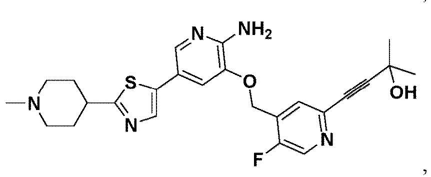 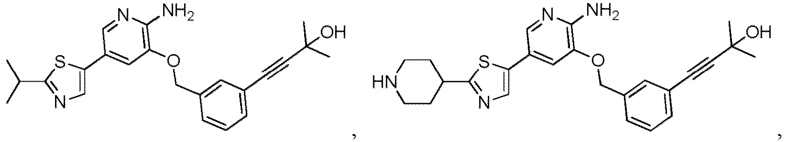 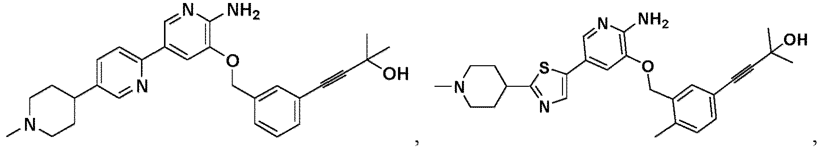 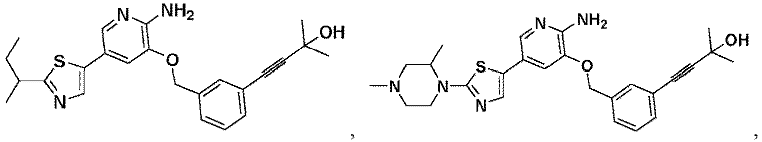 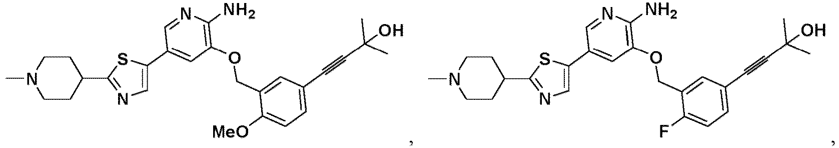 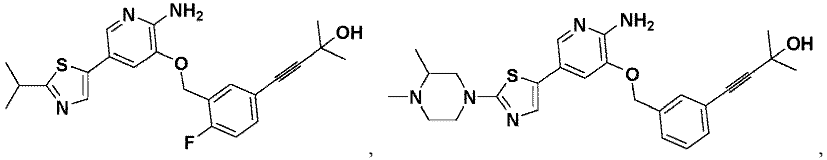 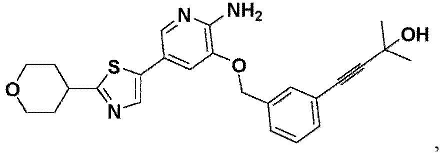 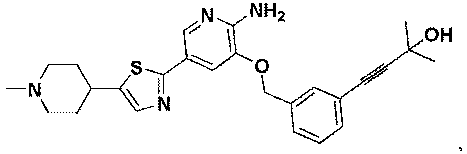 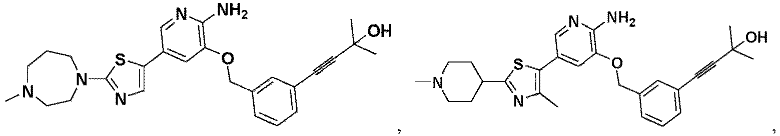 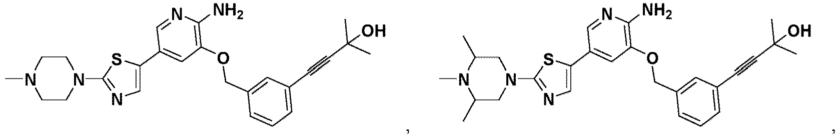 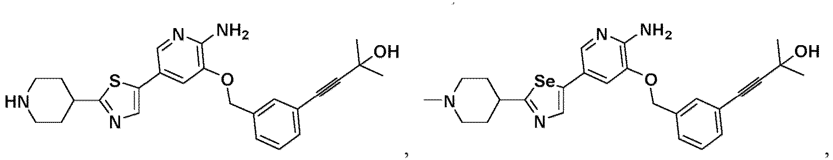 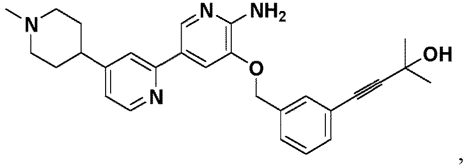 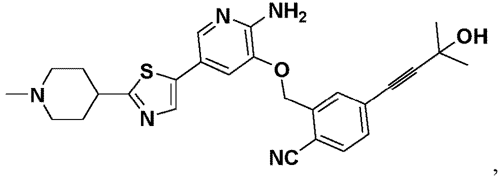 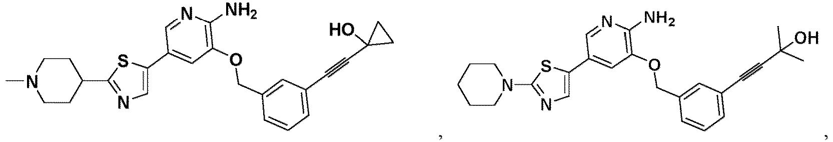 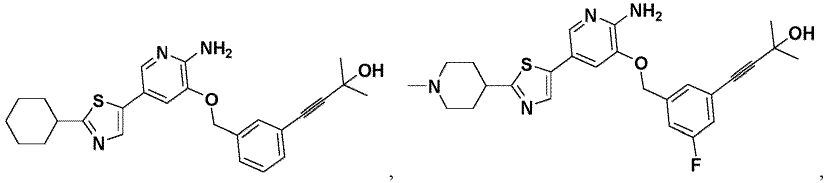 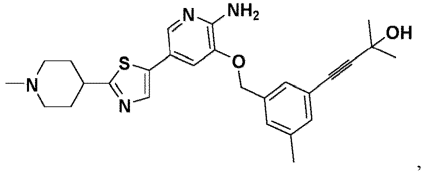 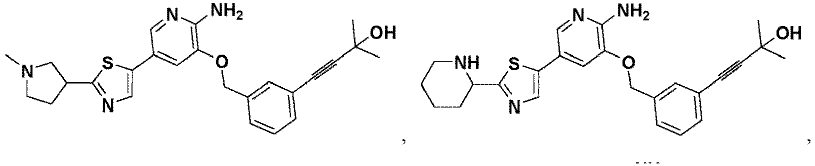 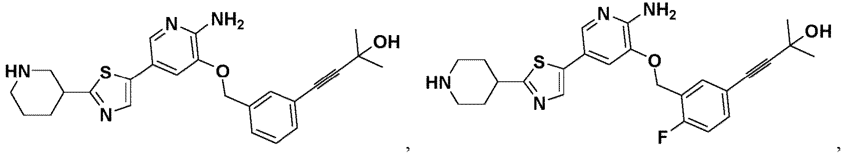 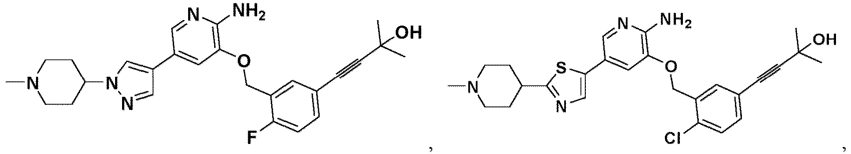 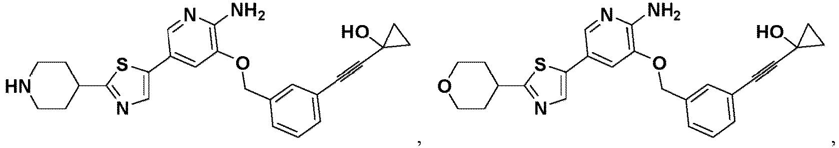 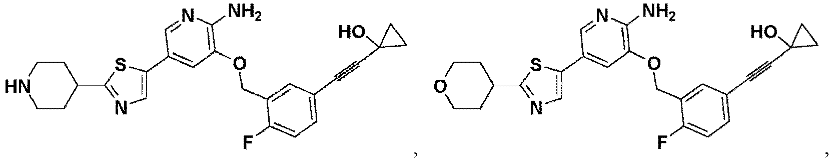 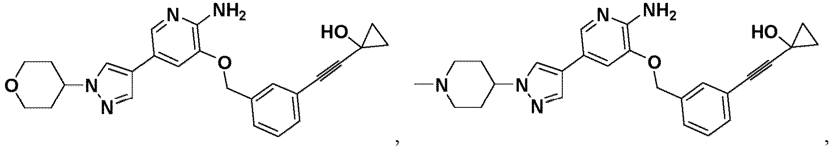 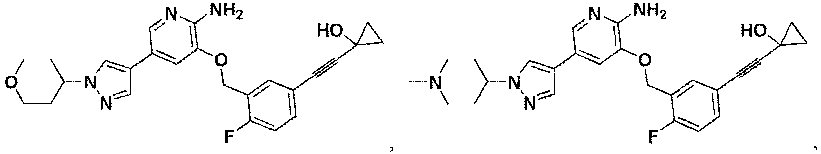 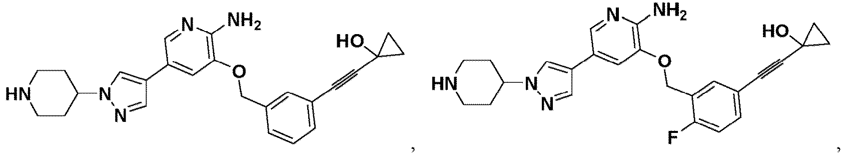 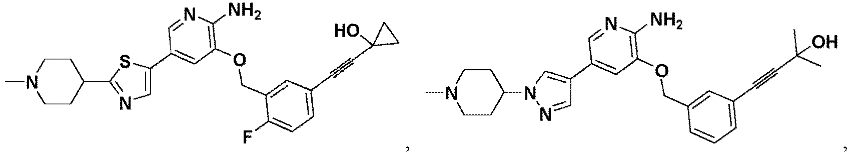 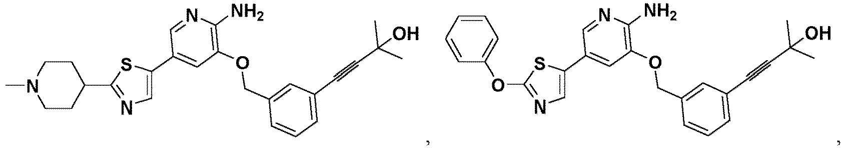 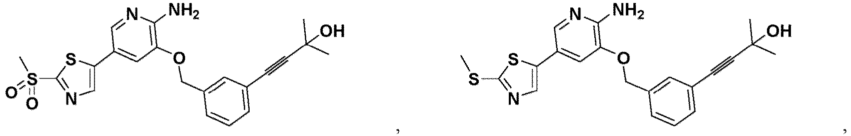 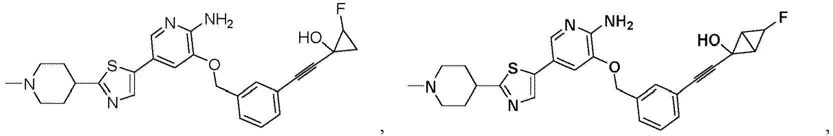 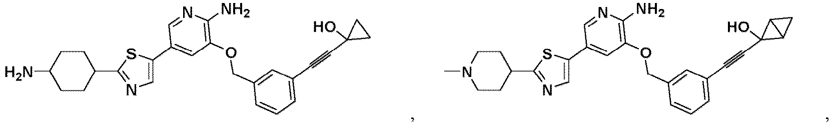 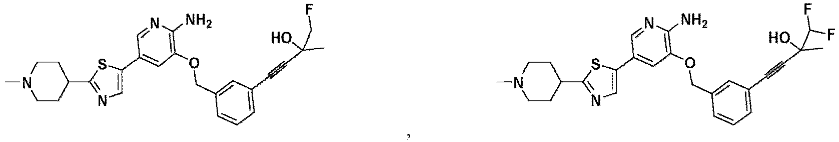 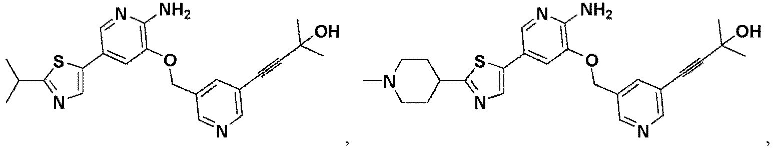 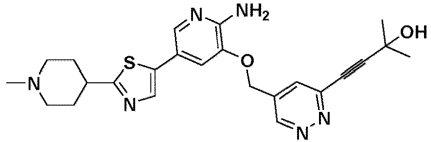 und

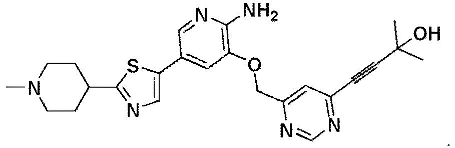

9. Verbindung zur Verwendung nach Anspruch 1, wobei das Coronavirus aus dem aviären infektiösen Bronchitis-Virus, dem Virus der übertragbaren Gastroenteritis des Schweins, der epizootischen Virusdiarrhö des Schweins, dem Virus dem hämagglutinierenden Enzephalomyelitis-Virus des Schweins, dem Maus-Hepatitis-Virus, dem Truthahn-Blaukamm-Virus, dem Rinder-Coronavirus, dem Coronavirus des Hundes, dem infektiösen Peritonitis-Virus der Katze, dem Coronavirus der Ratte, dem Sialodacryoadenitis-Coronavirus der Ratte und dem Coronavirus der epidemischen Diarrhö des Nerzes ausgewählt ist.

10. Verbindung zur Verwendung nach Anspruch 1, wobei das Coronavirus das infektiöse Peritonitis-Virus der Katze ist.

11. Verbindung zur Verwendung nach Anspruch 1, wobei das Tier ein Haustier ist.

12. Verbindung zur Verwendung nach Anspruch 1, wobei das Tier ein Begleittier oder ein Labortier ist.

13. Verbindung zur Verwendung nach Anspruch 1, wobei das Tier ein Nutzvieh oder ein Federvieh ist.

14. Verbindung zur Verwendung nach Anspruch 1, wobei die Krankheit aus der aviären infektiösen Bronchitis, der übertragbaren Gastroenteritis des Schweins, der epizootischen Diarrhö des Schweins, der Coronaviruserkrankung des Hundes, der hämagglutinierenden Enzephalomyelitis des Schweins, Hepatitis, Enzephalitis und Enteritis, verursacht durch das Maus-Hepatitis-Virus, Truthahnblaukamm, Diarrhö bei neugeborenen Kälbern, Rinderblut-Ruhr, infektiöse Peritonitis der Katze, Sialodacryoadenitis der Ratte und epidemische Diarrhöe des Nerzes.

15. Verbindung zur Verwendung nach Anspruch 1, wobei die Krankheit die infektiöse Peritonitis der Katze ist.

## Revendications

1. Composé, ou un sel pharmaceutiquement acceptable, un solvate ou un composé deutéré de celui-ci pour une utilisation dans la prévention et/ou le traitement d'une maladie causée par ou associée à une infection par un pathogène chez un animal ;
le composé possède la structure suivante : 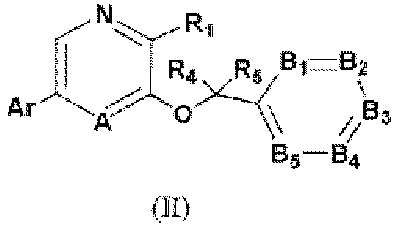 dans lequel,
A est sélectionné parmi C et N ;
Ar est sélectionné parmi : 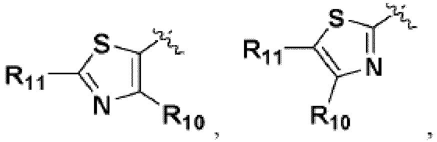 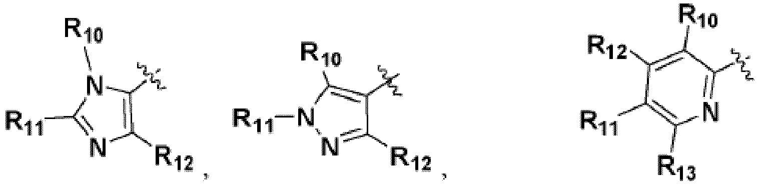 et 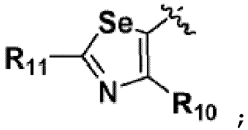
dans lequel R₁₀, R₁₂ et R₁₃ sont sélectionnés indépendamment parmi : -H, un alkyle linéaire/ramifié en C₁₋₁₀, un -N(alkyle en C₀₋₁₀) (alkyle en C₀₋₁₀), un -O-alkyle en C₀₋₁₀ et un cycloalkyle en C₃₋₁₀ ;
R₁₁ est sélectionné parmi : -H, un -O hétérocycloalkyle, un -N hétérocycloalkyle, un alkyle linéaire/ramifié en C₁₋₁₀, un cycloalkyle en C₃₋₁₀, un O-alkyle en C₀₋₁₀, un -N(alkyle en C₀₋₁₀) (alkyle en C₀₋₁₀), un -SO₂(alkyle en C₀₋₁₀), un - O(alkyle en C₀₋₁₀), un -O-phényle, un -S (alkyle en C₀₋₁₀), un -N hétérocycloaryle, un -O hétérocycloaryle et un -S hétérocycloaryle, dans lequel H sur l'atome C ou l'hétéroatome peut être remplacé par un alkyle linéaire en C₁₋₃, un -N(alkyle en C₀₋₁₀)(alkyle en C₀₋₁₀) ou -CF₃ ;
R₁ est sélectionné parmi : -H, un halogène, -NO₂, -CN, un alkyle linéaire/ramifié en C₁₋₅, un cycloalkyle en C₃₋₁₀, un -N(alkyle en C₀₋₁₀) (alkyle en C₀₋₁₀), -CF₃, -OCF₃, -OCHF₂, - OCH₂F et un -O-alkyle en C₀₋₁₀ ;
R₄ et R₅ sont sélectionnés indépendamment parmi : -H, un deutérium, un halogène, un alkyle linéaire/ramifié en C₁₋₁₀ et un alkyle linéaire/ramifié en C₁₋₁₀ deutéré ;
au moins un parmi B₁, B₂, B₃, B₄ et B₅ est N ; ou 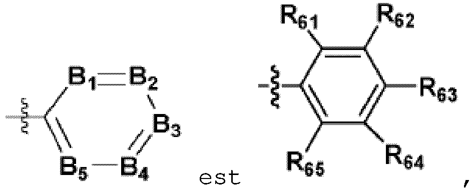 dans lequel
R₆₃ est sélectionné parmi : -H, un halogène, un -O-alkyle en C₀₋₁₀, un alkyle linéaire/ramifié en C₁₋₁₀, un -N(alkyle en C₀₋₁₀) (alkyle en C₀₋₁₀) et un cycloalkyle en C₃₋₁₀ ;
R₆₁, R₆₄ et R₆₅ sont sélectionnés indépendamment parmi : -H, un halogène, -CN, un -O-alkyle en C₀₋₁₀, un alkyle linéaire/ramifié en C₁₋₁₀, un -N(alkyle en C₀₋₁₀) (alkyle en C₀₋₁₀), un cycloalkyle en C₃₋₁₀, -C≡C-R₇, un -O-hétérocycloalkyle, un -N-hétérocycloalkyle et un alkyle linéaire/ramifié en C₁₋₅ contenant O et N ;
R₆₂ est C≡C-R₇ ;
R₇ est sélectionné parmi H, un alkyle linéaire/ramifié en
C₁₋₅, un cycloalkyle en C₃₋₁₀ et 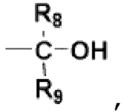 dans lequel R₈ et
R₉ sont sélectionnés indépendamment parmi : -H, -CF₃, -CHF₂, -CH₂F, un alkyle linéaire/ramifié en C₁₋₁₀, un -CH=C(alkyle en C₀₋₁₀) (alkyle en C₀₋₁₀), un -C≡C(alkyle en C₀₋₁₀), un cycloalkyle en C₃₋₁₀, un -CO(alkyle en C₀₋₁₀)(alkyle en C₀₋₁₀), un -CO(alkyle en C₀₋₁₀)(alkyle en C₀₋₁₀), un -CON(alkyle en C₀₋₁₀)(alkyle en C₀₋₁₀), un -N(alkyle en C₀₋₁₀)CO(alkyle en C₀₋₁₀)(alkyle en C₀₋₁₀), un groupe cyclique aromatique à cinq chaînons et un groupe cyclique aromatique à six chaînons, ou R₈ et R₉, conjointement avec les atomes de carbone entre R₈ et R₉, forment un cycloalkyle en C₃₋₈ ou un hétérocycloalkyle en C₃₋₈ contenant -O- ou -S-, un cycloalkyle condensé en C₄₋₉, un spirocycloalkyle en C₅₋₁₀, un cycloalkyle ponté en C₄₋₉, un cyclolactame en C₃₋₇, une lactone cyclique en C₃₋₇ ou une cétone cyclique en C₃₋₇ ; et
le pathogène est un coronavirus.

2. Composé pour l'utilisation selon la revendication 1, dans lequel R₁₁ est sélectionné parmi :

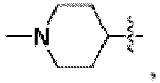

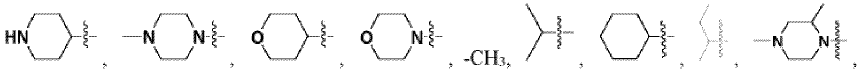

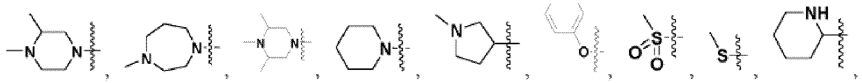

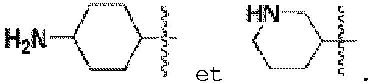

3. Composé pour l'utilisation selon la revendication 1, dans lequel R₆₃ est sélectionné parmi : -H, un halogène, un -O-alkyle en C₀₋₁₀ et un alkyle linéaire/ramifié en C₁₋₁₀.

4. Composé pour l'utilisation selon la revendication 1, dans lequel R₆₃ est sélectionné parmi : -H, -F et -OCH₃.

5. Composé pour l'utilisation selon la revendication 1, dans lequel R₆₁, R₆₄ et R₆₅ sont sélectionnés indépendamment parmi : -H, un halogène, un alkyle linéaire/ramifié en C₁₋₃, un -O-alkyle en C₀₋₃ et un alkyle linéaire/ramifié en C₁₋₃ contenant N.

6. Composé pour l'utilisation selon la revendication 1, dans lequel R₆₁, R₆₄ et R₆₅ sont sélectionnés indépendamment parmi : -H, -F, -Cl, -CH₃, -CH₂NH₂, -CN et -OCH₃.

7. Composé pour l'utilisation selon la revendication 1, dans lequel R₈ et R₉ sont sélectionnés indépendamment parmi : -H, -CF₃, -CHF₂, -CH₂F, -CH₃, -CH₂CH₃, -CH=CH₂, 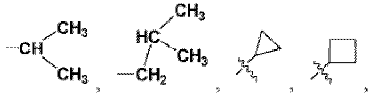 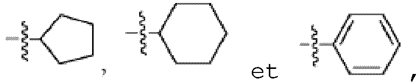 ou R₈ et R₉, conjointement avec les atomes de carbone entre R₈ et R₉, forment

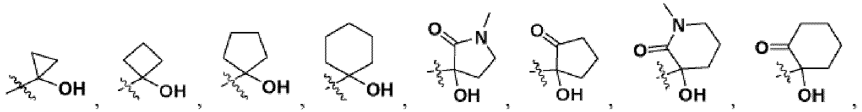

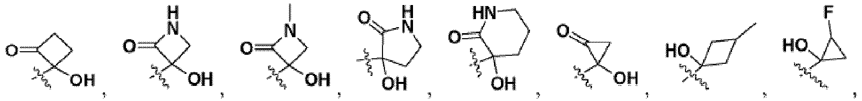

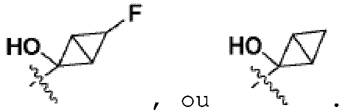

8. Composé pour l'utilisation selon la revendication 1, dans lequel le composé est sélectionné parmi :

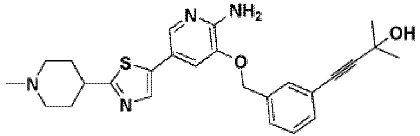

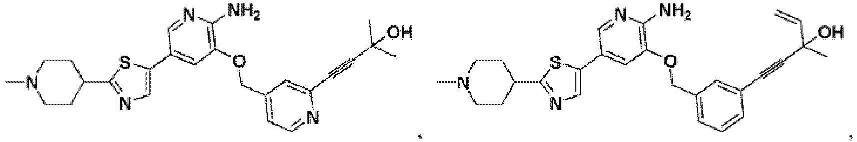

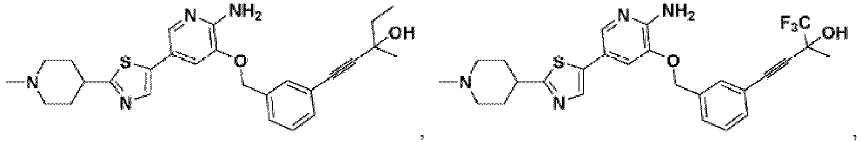

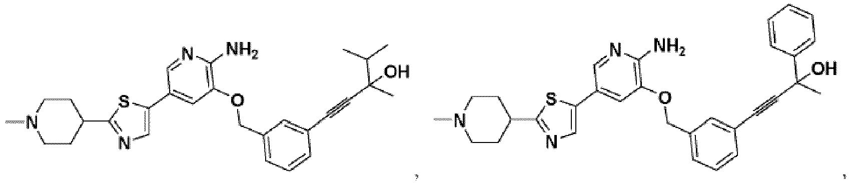

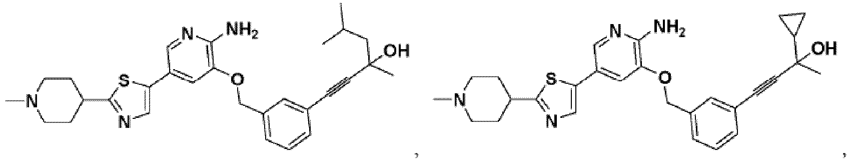

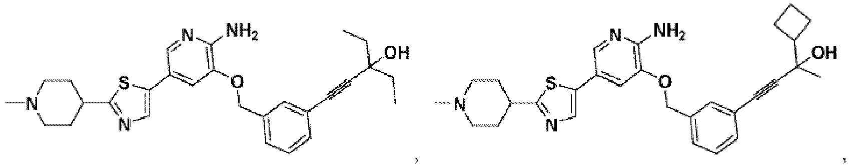

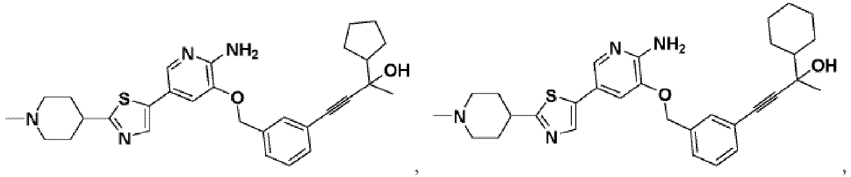

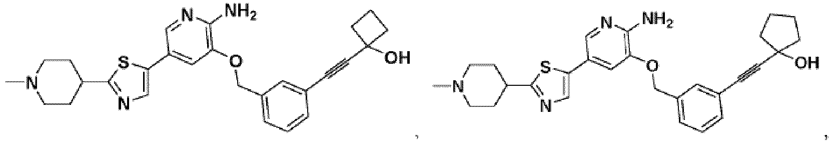

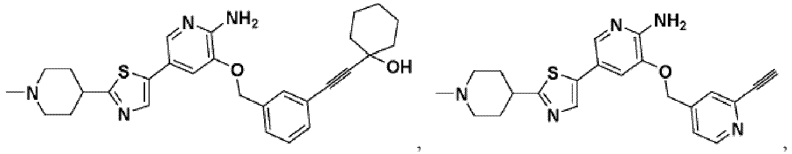

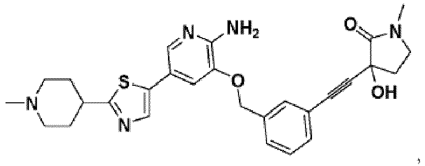

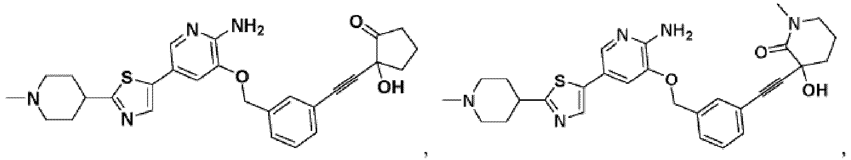

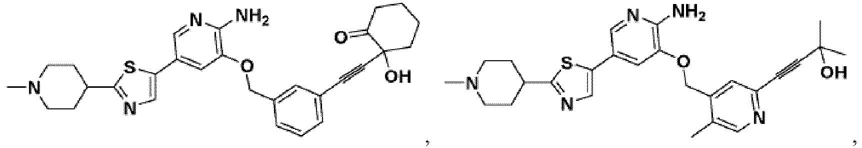

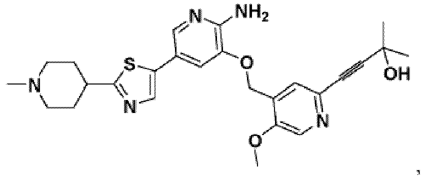

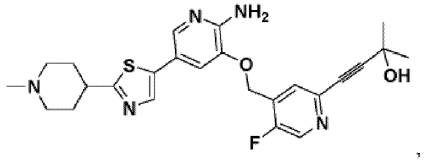

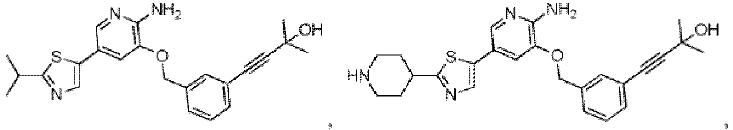

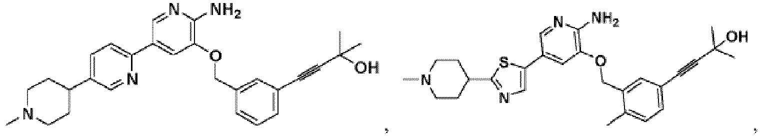

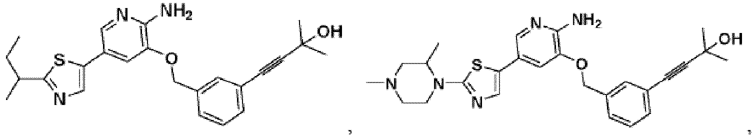

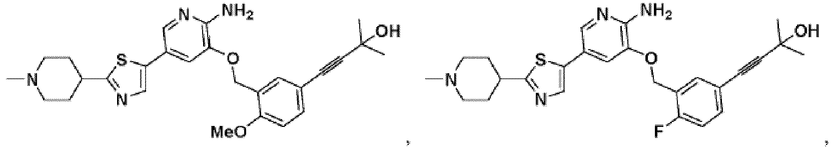

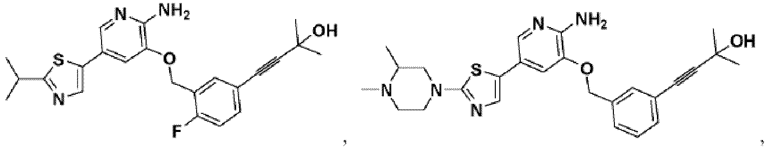

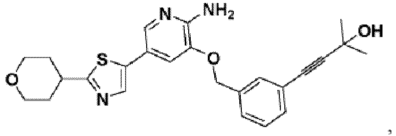

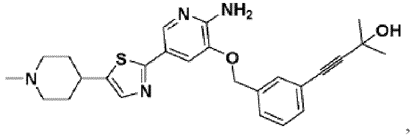

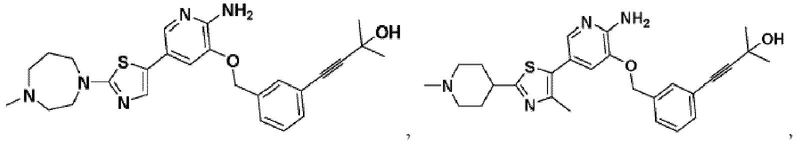

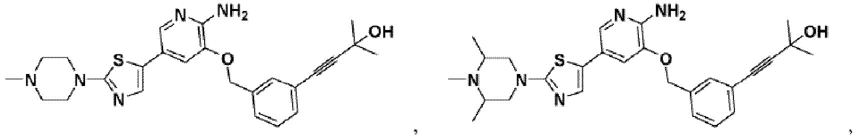

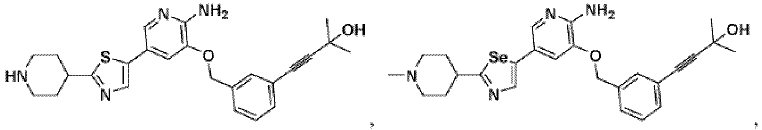

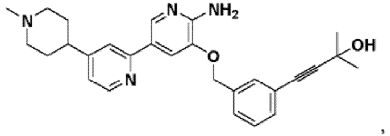

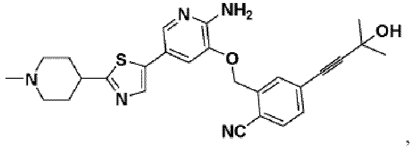

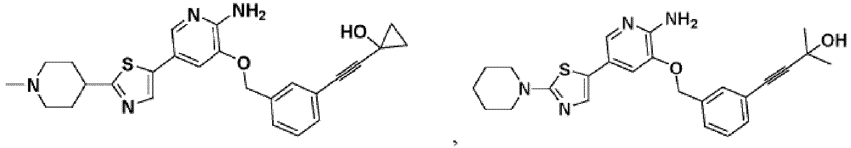

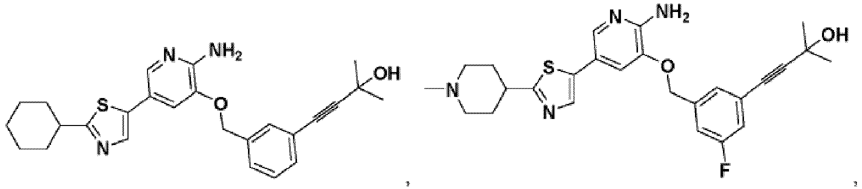

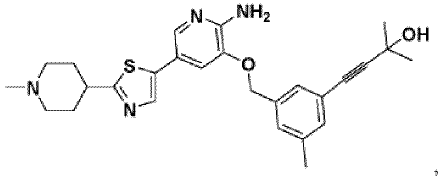

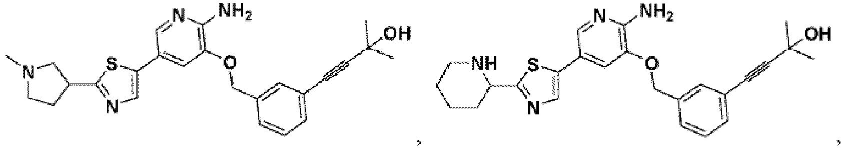

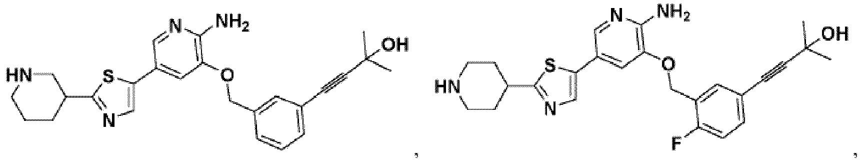

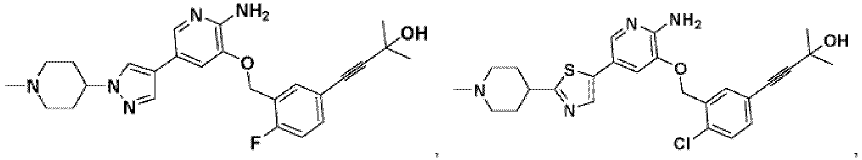

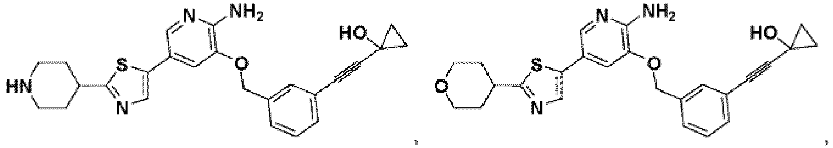

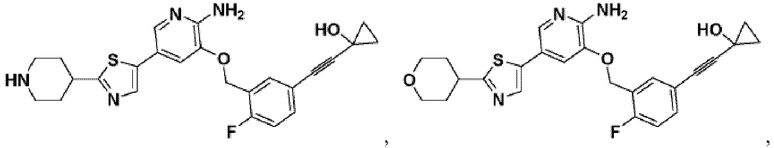

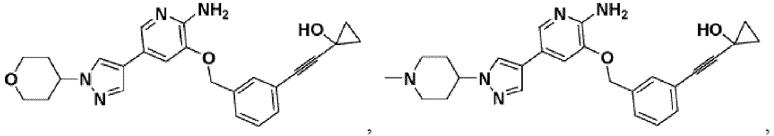

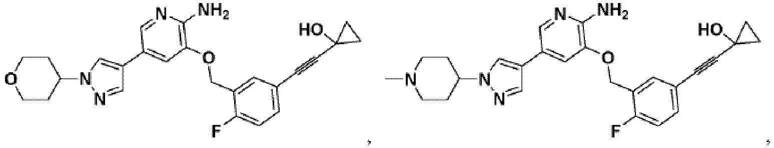

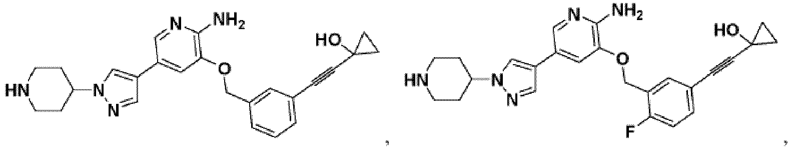

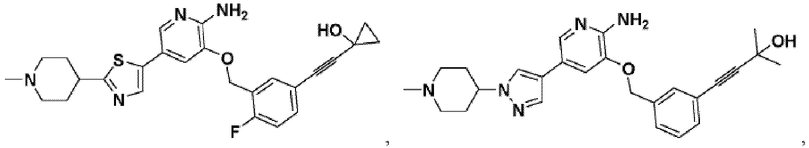

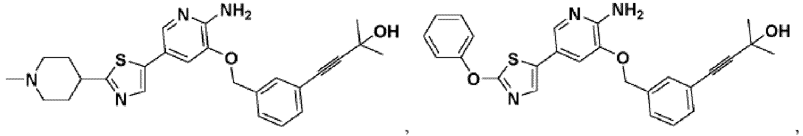

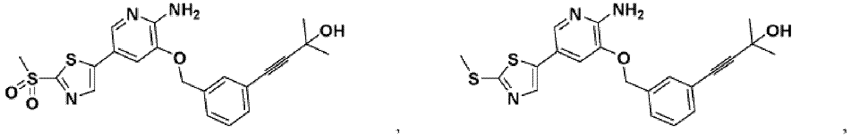

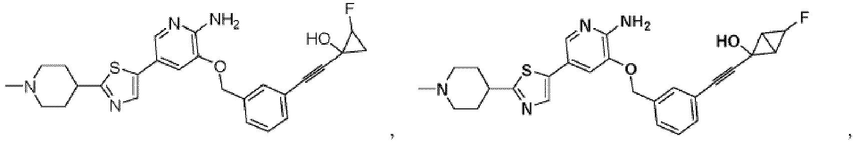

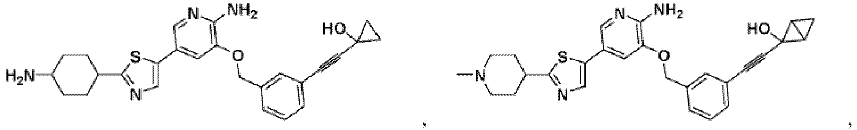

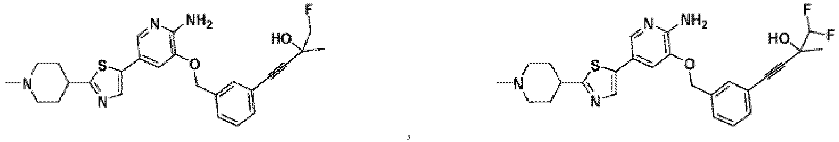

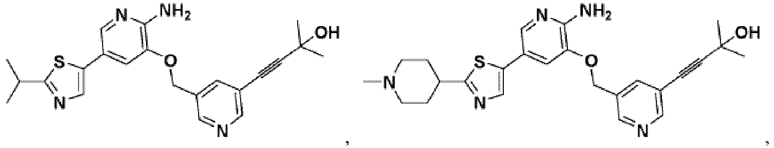

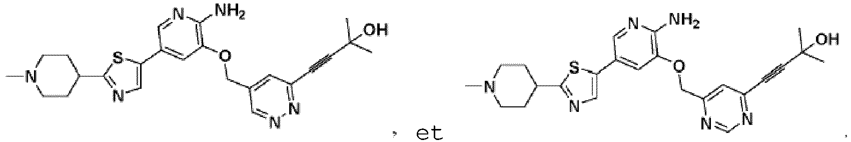

9. Composé pour l'utilisation selon la revendication 1, dans lequel le coronavirus est sélectionné parmi le virus de la bronchite infectieuse aviaire, le virus de la gastroentérite transmissible porcine, le virus de la diarrhée épidémique porcine, le virus de l'encéphalomyélite hémagglutinante porcine, le virus de l'hépatite de la souris, le virus de la crête bleue de la dinde, le coronavirus bovin, le coronavirus canin, le virus de la péritonite infectieuse féline, le coronavirus du rat, le coronavirus de la sialodacryoadénite du rat et le coronavirus de la diarrhée épidémique du vison.

10. Composé pour l'utilisation selon la revendication 1, dans lequel le coronavirus est le virus de la péritonite infectieuse féline.

11. Composé pour l'utilisation selon la revendication 1, dans lequel l'animal est un animal domestique.

12. Composé pour l'utilisation selon la revendication 1, dans lequel l'animal est un animal de compagnie ou un animal de laboratoire.

13. Composé pour l'utilisation selon la revendication 1, dans lequel l'animal est du bétail ou de la volaille.

14. Composé pour l'utilisation selon la revendication 1, dans lequel la maladie est sélectionnée parmi la bronchite infectieuse - aviaire, la gastroentérite transmissible porcine, la diarrhée épidémique porcine, la maladie à coronavirus canine, l'encéphalomyélite hémagglutinante porcine, l'hépatite, l'encéphalite et l'entérite causées par le virus de l'hépatite de la souris, la crête bleue de la dinde, la diarrhée du veau néonatal, la dysenterie sanguine bovine, la péritonite infectieuse féline, la sialodacryoadénite du rat et la diarrhée épidémique du vison.

15. Composé pour l'utilisation selon la revendication 1, dans lequel la maladie est la péritonite infectieuse féline.
